# EUROPEAN PATENT APPLICATION

(11) **EP 3 628 676 A1**
(43) Date of publication of application: **01.04.2020**
(21) Application number: 18306284.3
(22) Date of filing: 28.09.2018
(51) Int. Cl.: C07K 5/02, C07D 513/00, C07D 513/12, C07K 7/02, C07D 513/22, A61K 38/00, A61K 31/00

(54) **MODIFIED PEPTIDES AND THEIR USES**

(71) Applicant: Université de Montpellier, 34090 Montpellier (FR); Centre National de la Recherche Scientifique, 75016 Paris (FR); Ecole Nationale Supérieure de Chimie de Montpellier, 34090 Montpellier (FR); Institut de Recherche pour le Developpement, 34394 Montpellier (FR); Centre Hospitalier Universitaire de Montpellier, 34295 Montpellier Cedex 05 (FR)
(72) Inventor: BONNEL, Clément, Nottingham, Nottinghamshire NG1 7JF (GB); LEGRAND, Baptiste, 34093 Montpellier (FR); MAILLARD, Ludovic, 34380 Causse-de-la-Selle (FR); MASURIER, Nicolas, 34070 Montpellier (FR); MARTINEZ, Jean, 34720 Caux (FR); LICZNAR-FAJARDO, Patricia, 34725 Saint-andré-de-sangonis (FR); JUMAS-BILAK, Estelle, 30190 Sauzet (FR)
(74) Representative: LLR

(57) **Abstract**

The invention relates to a compound of formula A 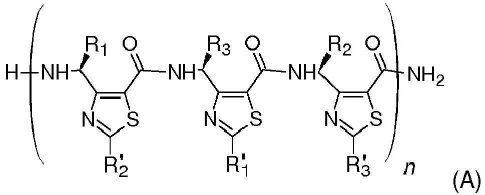 Wherein n is an integer from 1 to 6, and R₁, R₁', R₂, R₂', R₃, R₃' are cationic or hydrophobic residues.

## Description

The invention relates to modified peptides and their use

The rapid expansion of drug-resistant infections has presented a serious challenge to conventional antimicrobial therapies. As examples, methicillin-resistant *Staphylococcus aureus,* ampicillin-resistant *Escherichia coli* and vancomycine-resistant *enterococci* have arisen as common nosocomial germs. In such a context, antimicrobial peptides (AMPs) provide opportunities to develop new generation of antibiotics. Today few thousand AMPs displaying a broad-spectrum bactericidal activity have been isolated from all kingdom of life including bacteria, archea and eukaryotes. In fungi, plants and animals, the host-defense peptides play a central role in the innate immunity system.[M. Zasloff, Nature 2002, 415, 389-395; R. E. Hancock, G. Diamond, Trends Microbiol. 2000, 8, 402-410] AMPs are typically 12 to 80 residues in length and span several secondary structures including α-sheet, β-helix, extended and loop. Despite a structural heterogeneity, most common AMPs present similar features. The great majority of AMPs are poly-cationic sequences and the antimicrobial activity results from spatial segregation of the cationic and hydrophobic side chains onto distinctly regions or faces of the molecule. These features together appear to be crucial to initiate electrostatic interactions with the negatively charged components of the bacterial envelope, i.e. lipopolysaccharides and techoic acids as well as with the negatively charged phospholipids of the bacteria membrane. After reaching some threshold concentration, AMPs may self-assemble on the membrane surface. Therefore, hydrophobic interactions allow the peptides to insert into the phospholipid bilayer leading to depolarization or disruption of the bacterial cell wall. Although other mechanism of action have been proposed, it is widely believed that such a simple physicochemical destabilization of the membrane results in a relatively low propensity to elicit bacterial resistance.

Nevertheless, despite promising results in early-stage clinical trials, most AMPs have faced some difficulties for practical applications. These failures are mainly linked to the poor metabolic stability and hemolytic activity of the peptides.

By using the positively charged amphipathic features as templates, synthetic AMP analogues have been constructed to maximize antimicrobial activity while also minimizing hemolytic activity. One method was to modify existing AMP sequences to optimize therapeutic potential. In a second approach it has been proposed to tailor the physicochemical characteristics of AMPs onto new classes of non-natural polymers.

Peptidomimetic antimicrobials have first been made using β-peptides.[Y. Hamuro, J. P. Schneider, W. F. DeGrado, J. Am. Chem. Soc. 1999, 121, 12200-12201; D. Liu, W. F. DeGrado, J. Am. Chem. Soc. 2001, 123, 7553-7559; E. A. Porter, X. Wang, H. S. Lee, B. Weisblum, S. H. Gellman, Nature 2000, 404, 565; T. L. Raguse, E. A. Porter, B. Weisblum, S. H. Gellman, J. Am. Chem. Soc. 2002, 124, 12774-12785; E. A. Porter, B. Weisblum, S. H. Gellman, J. Am. Chem. Soc. 2002, 124, 7324-7330; P. I. Arvidsson, N. S. Ryder, H. M. Weiss, D. F. Hook, J. Escalante, D. Seebach, Chem. Biodiv. 2005, 2, 401-420.] These molecules folded into amphipathic structures demonstrated potent in vitro anti-bacterial activity. In addition, unlike the α-peptide backbone of natural AMPs, they offer the additional advantage of being resistant to proteases. However, as for many AMPs,[A. K. Marr, W. J. Gooderham, R. E. Hancock, Curr. Opin. Pharmacol. 2006, 6, 468-472; M. Vaara, Curr Opin Pharmacol 2009, 9, 571-576.] the first generation compounds have low discrimination for bacterial versus mammalian cells resulting in toxic side effects such as hemolysis.[Y. Hamuroet al., J. Am. Chem. Soc. 1999, 121, 12200-12201*]*

It is now recognized that parameters influencing both selectivity and affinity for bacteria include chain length, net charge and hydrophobic/hydrophilic balance of the oligomers. While preorganization of the backbone is not crucial since the formation of the global amphipathic structure may occur upon interactions with bacterial membranes, oligomers folding into well-defined secondary structures and known as foldamers have been preferentially used to template the facially amphiphilic morphology of natural AMPs.

Thus, in line of the success of β-peptides, peptoids [J. A. Patch, A. E. Barron, J. Am. Chem. Soc. 2003, 125, 12092-12093], oligo-ureas [A. Violette, et al., Chem. Biol. 2006, 13, 531-538*;* P. Claudon, et al., Angew. Chem., Int. Ed. Engl. 2010, 49, 333-336], arylamides [G. N. Tew, et al., PNAS 2002, 99, 5110-5114*;* D. Liu, et al., Angew Chem Int Ed Engl 2004, 43, 1158-1162*;* S. Choi, et al., PNAS 2009, 106, 6968-6973] or arylureas *[*J. Hua, et al., Mol. Oral. Microbiol. 2010, 25, 418-425*;* Hua, et al., Mol. Oral. Microbiol. 2010, 25, 426-432*;* H. Tang, R. Et al., Chem. Commun. 2005, 1537-1539.] and phenylene ethylenes [L. Arnt et al., J. Polym. Scie. Part A: Polym. Chem. 2004, 42, 3860-3864] have been used as scaffold. In particular, Brilacidin (PMX-30063) an arylamide foldamer is currently in phase II human clinical trial, demonstrating the therapeutic potential of AMPs-mimicking antibiotic agents.

Thus, one aim of the invention is to propose new polycationic molecular pseudo-peptide architectures.

Another aim of the invention is to provide new therapeutic applications of the above compounds.

The invention thus relates to a compound of formula A : 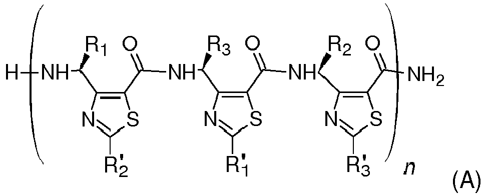
wherein n is an integer from 1 to 6,
wherein R₁ and R₁' are both either cationic residues or hydrophobic residues, said residues being identical or different,
wherein R₂ and R₂' are both either cationic residues or hydrophobic residues, said residues being identical or different; and
Wherein R₃ and R₃' are both either cationic residues or hydrophobic residues, said residues being identical or different;
wherein
   - when simultaneously R₁ and R₁' are cationic residues, then R₂, R₂', R₃ and R₃' are hydrophobic residues;
   - when two simultaneously R₂ and R₂' are cationic residues, then R₁, R₁', R₃ and R₃' are hydrophobic residues; and
   - when two simultaneously R₃ and R₃' are cationic residues, then R₁, R₁', R₂ and R₂' are hydrophobic residues,
or a salt or solvate, or its enantiomer thereof.

This invention is based on the unexpected observation made by the inventors that an artificial peptide according to formula A is an efficient antibacterial compound.

The compounds according to the inventions harbor, as demonstrated in the Examples, antibacterial properties, due to their specific structure.

As mentioned above, the compound according to the invention is a trimer, each unit of which being substituted by either cationic residues or hydrophobic residues. The spatial organization and distribution of these residues is essential for the activity of the compounds.

R residues (i.e. R₁, R₂ and R₃) and their corresponding R' residues (respectively R'₁, R'₂ and R'₃) have to be of the same nature, i.e. cationic of hydrophobic, but can be structurally different. For instance, if R₁ is an hydrophobic linear alkyl, R'₁ will be an hydrophobic residue that could be an aryl. Also, if R₁ is cationic primary amine, R'₁ will be a cationic residue that could be an a secondary or a tertiary amine.

Another important aspect of the compound according to the invention is the fact that only one R residue and its corresponding R' residue can be cationic residues. This means that the two remaining couple of residues R;R' would be hydrophobic residues.

A solvate of the above compounds, may comprise a stoichiometric or non-stoichiometric amount of a solvent bound by non-covalent intermolecular forces. Preferred is a solvent which is non-volatile, non-toxic, and suitable for administration to humans. Ethanol, methanol, propanol, and methylene chloride may be used.

A salt of the above compounds may be any anionic salts such as chloride, bromide, sulfate, nitrate, phosphate, (bi-)carbonate, acetate, propionate, glycolate, pyruvate, oxalate, malate, malonate, succinate, fumarate, tartrate, citrate, benzoate, cinnamate, mandelate, methanesulfonate, ethanesulfonate, p-toluene-sulfonate, salicylate salts ...

Salts of the above compounds may be prepared from inorganic and organic acids. Salts derived from inorganic acids include hydrochloric acid, hydrobromic acid, sulfuric acid, nitric acid, phosphoric acid, and the like. Salts derived from organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

The compound of formula A above-mentioned can be modified, in particular to have the following formula A1 when acetylated :

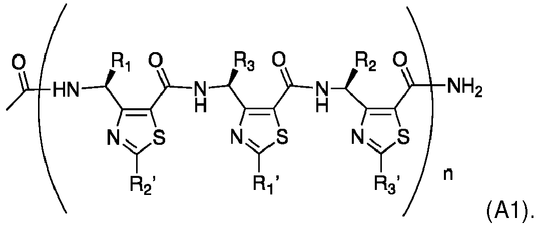

The compounds according to the invention displays a facially amphiphilic/amphipatic helical molecular conformation.

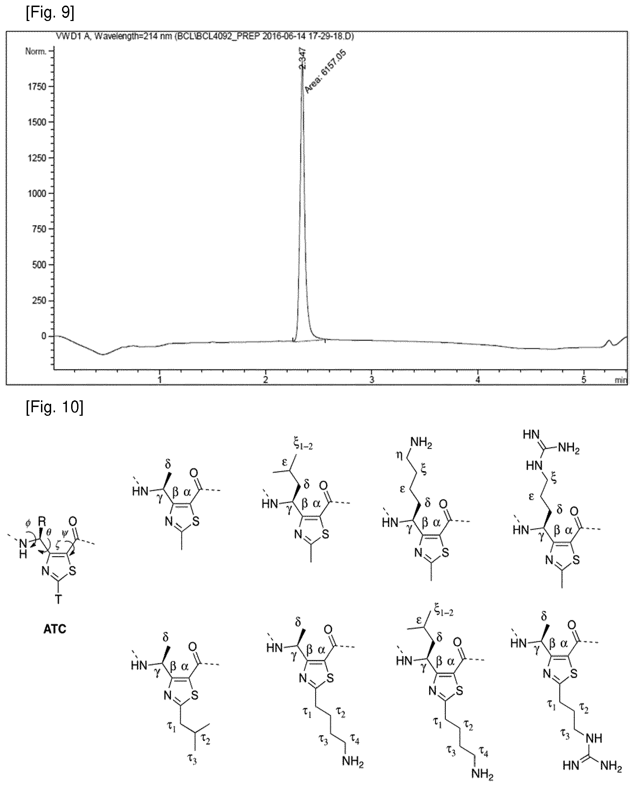

"Helical molecular conformation" means that the compounds defined above adopt a repetitive secondary structure, when n is higher than 1, in which the relationship of one γ-aminoacid unit to the next is the same for all alpha-carbons, all beta-carbons and all gamma-carbons. This means that the dihedral angles phi, theta, zeta and psi are similar for each γ-aminoacid in the helical confomation. Phi, theta, zeta and psi (**Fig. 10**) average values can be -72±40, 114±40, 0±10, -23±30 respectively. Phi, theta, zeta and psi average values can also be 72±40, -114±40, 0±10, 23±30 respectively. The conformation is stabilized by a hydrogen-bonding pattern between the carbonyl group of one γ aminoacid unit and the NH of the next γ-aminoacid unit.

The positioning of the substituents defined above as R1, R1', R2, R2', R3, R3' defines three distinct faces over the γ-peptide helix. Substituents R1, R1' as defined above share a first face. Substituents R2, R2' as defined above share a second face. Substituents R3, R3' as defined above share a third face.

" Facially amphiphilic/amphipatic" means that the cationic and hydrophobic substituents are distributed on different faces along the long axis of the helix.

Advantageously, the invention relates to the above mentioned compounds, wherein said hydrophobic residues are chosen from the group consisting of : C₁-C₁₆ linear, branched or cyclic alkyl and C₄-C₁₆ aryl or arylalkyl, substituted of not, branched or not, or C₄-C₁₆ heteroaryl, or heteroarylaklyl substituted of not, branched or not.

The hydrophobic residues are advantageously C₁-C₁₆ linear, branched or cyclic alkyl which means that the residues can be the following groups : methyl, ethyl, propyl, isopropyl, n-butyl, sec-butyl, iso-butyl, tert-butyl, cyclobutyl, n-pentyl, tert-pentyl, neopentyl, isopentyl, sec-pentyl, 3-pentyl, sec-isopentyl, cyclopentyl, n-hexyl, 2,2-dimethylbutyl, 2,3-dimethylbutyl, 2-methypentyl, 3-methylpentyl, cyclohexyl, methylcyclopentyl, n-heptyl, 2-methylhexyl, 3-methylhexyl, 2,2-dimethylpentyl, 2,3-dimethylpentyl, 2,4-dimethylpentyl, 3,3-dimethylpentyl, 3-ethylpentyl, 2,2,3-triméthylbutyl, cyclohexyl, 2-methylcyclohexyl, 3-methylcyclohexyl, 4-methylcychohexyl, 2,3-dimethylcyclopentyl, 2,4-dimetylcyclopentyl, 2,5-dimethylcyclopentyl, 3,4-dimethylcyclopentyl, 2-ethylcyclomentyl,.

C₄-C₁₆ aryl or arylalkyl, substituted of not, branched or not, or C₄-C₁₆ heteroaryl, or heteroarylaklyl substituted of not, branched or not according to the invention can be the following groups : phenyl, 2-methylphenyl, 3-methylphenyl and 4-methylphenyl.

Other examples of hydrophobic residues are the following ones :

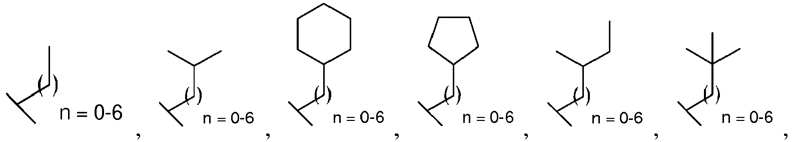

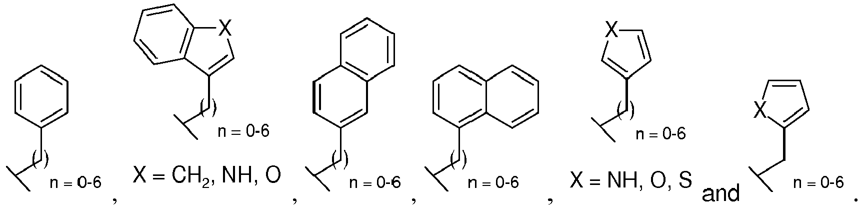

Advantageously, the invention relates to the compound as defined above, wherein said cationic residue is a -(CH₂)ₘ₋R₄ residue, m being an integer from 1 to 7, wherein R₄ is an amino, an amido, an amidino, a guanino, a guanidino, an imino or a pyridino residue.

More advantageously, the invention relates to the compound as defined above, wherein when R₁ and R₁', or R₂ and R₂', or R₃ and R₃' are cationic residues, said residues being identical.

In this advantageous embodiment of the invention, a couple of R,R' cationic residues are of the same nature, and advantageously, are the same -(CH₂)ₘ₋R₄ residues as defined above.

Other cationic residues can be the following ones: 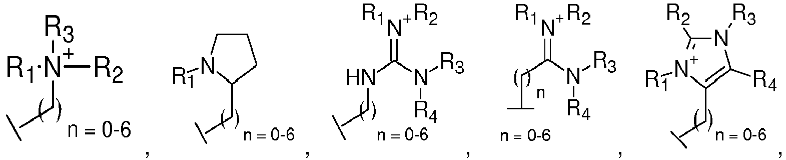 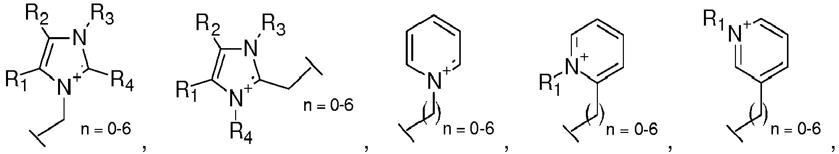 and 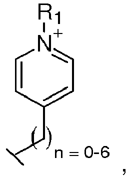 wherein independently from each other R₁, R₂, R₃ and R₄ can be H, an alkyl, an aryl or a heteroaryl.

Advantageously, the invention relates to the compound above defined, said compound being chosen from the compounds of the group consisting of : 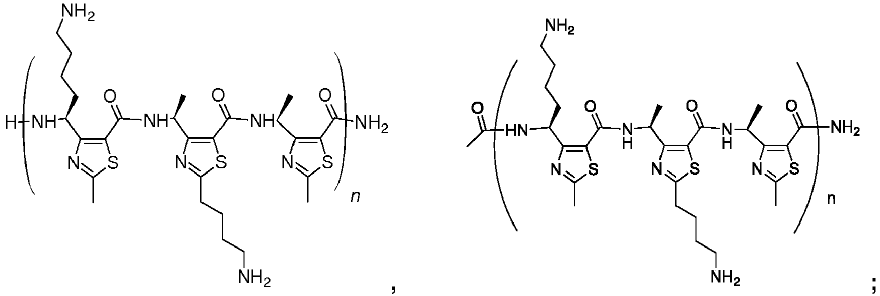 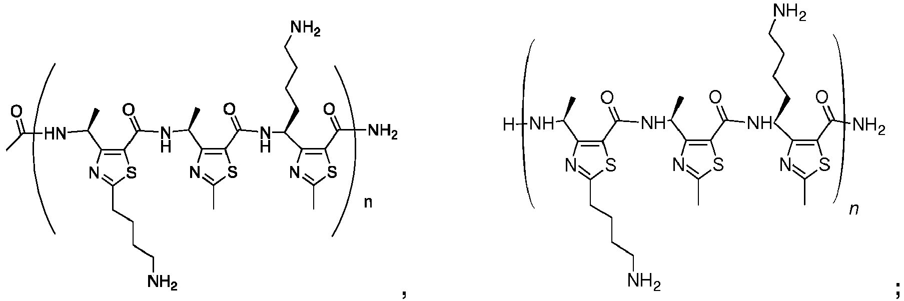 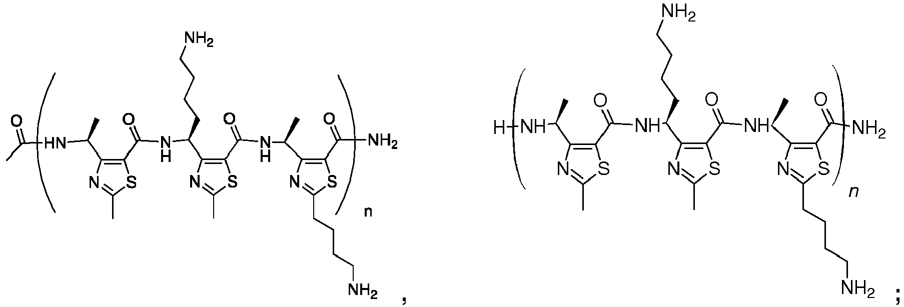 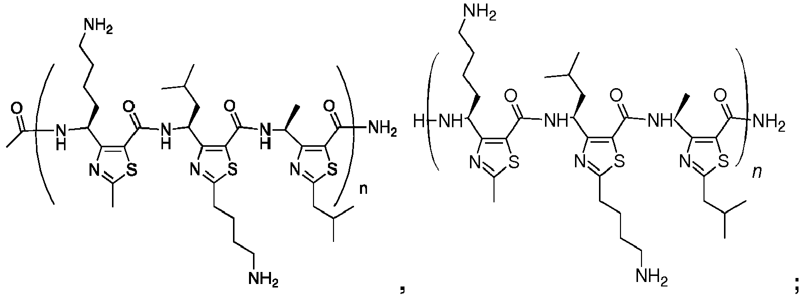 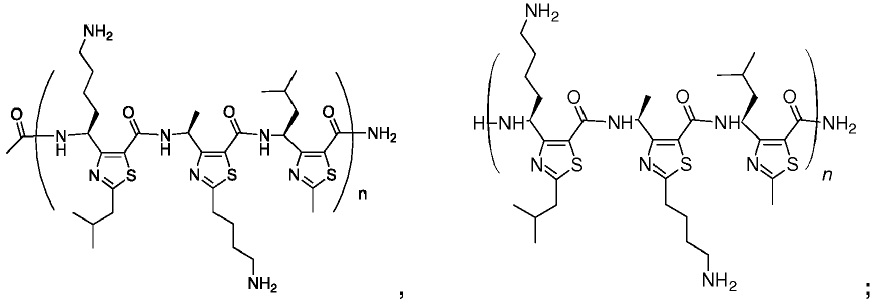 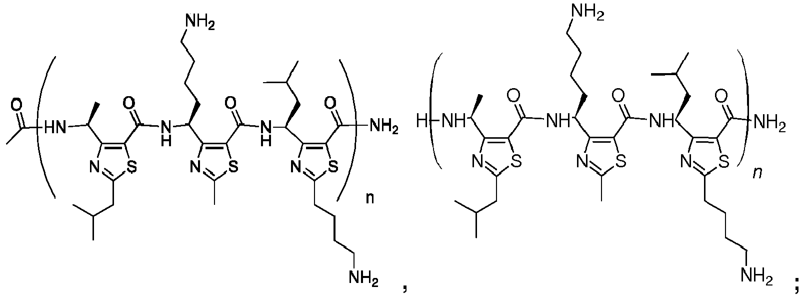 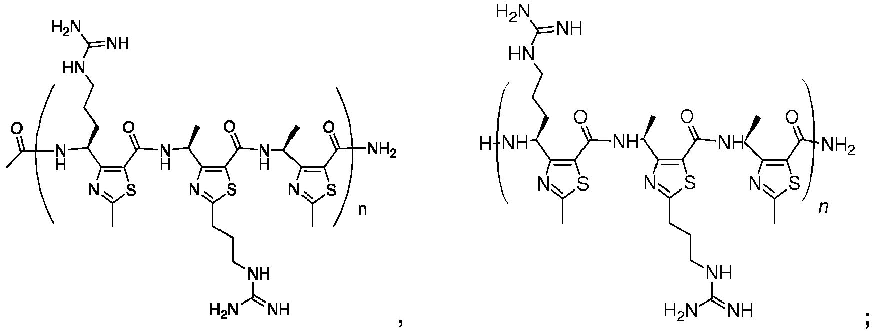 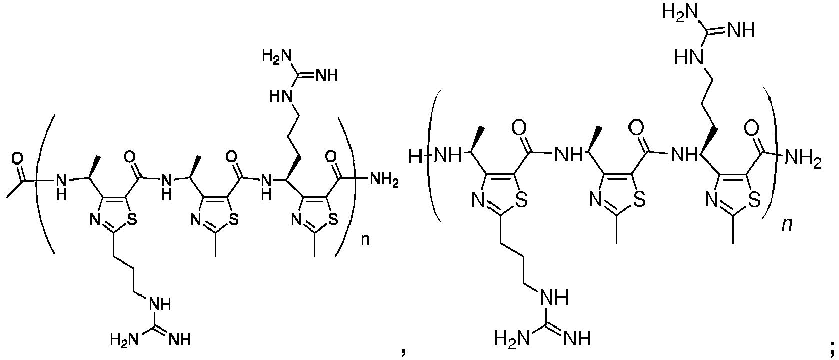 and 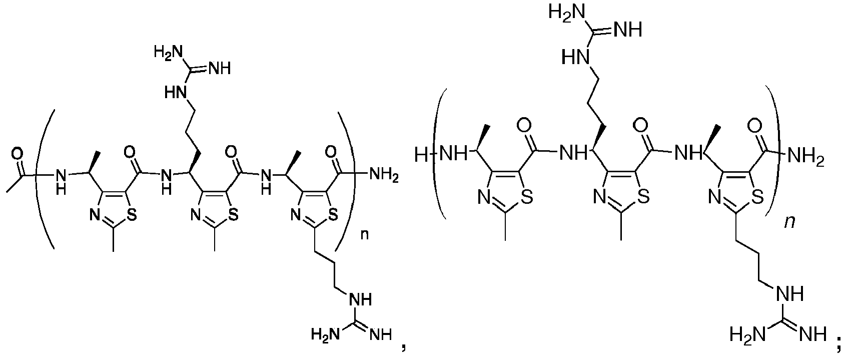 wherein n is an integer from 1 to 4.

Another advantageous embodiment, the invention relates to the compound defined above, wherein said compound is chosen from the compounds of the group consisting of :

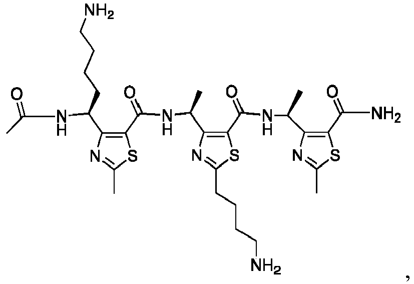

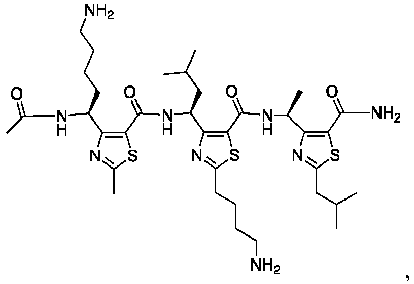

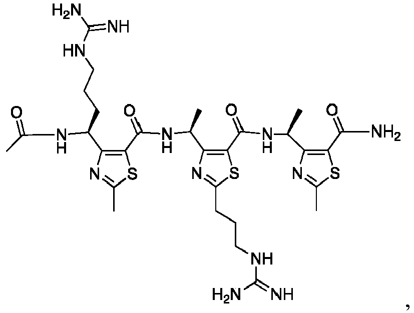

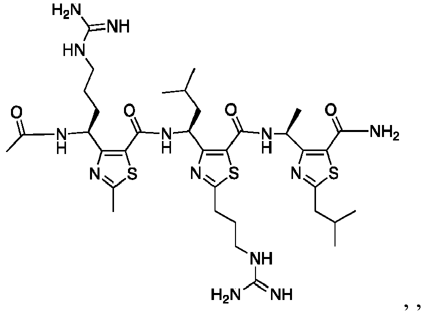

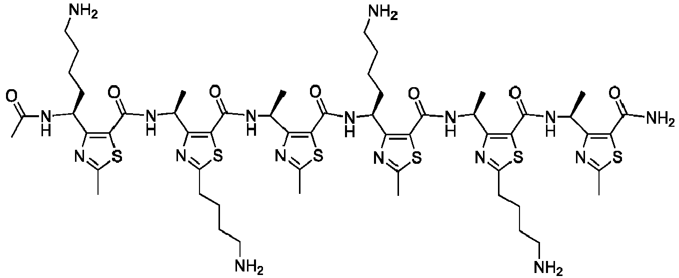

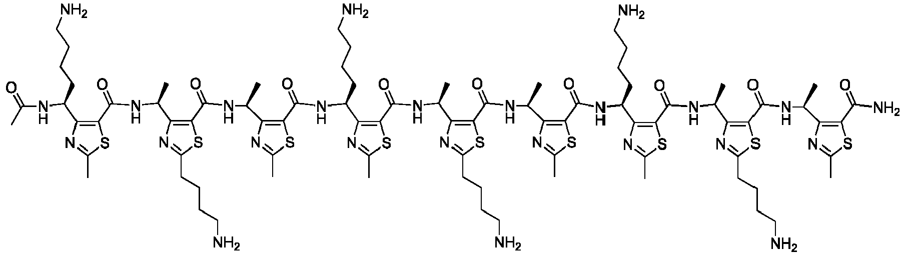

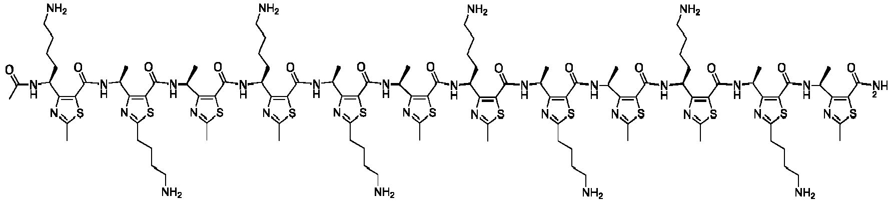

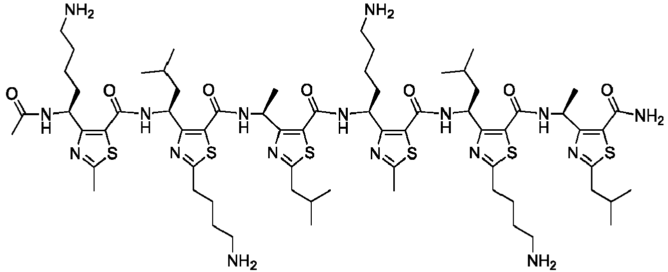

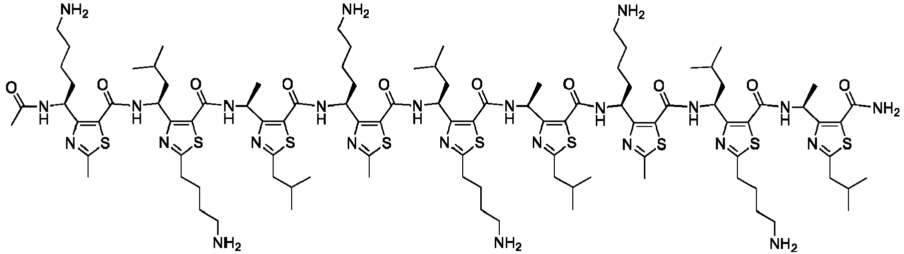

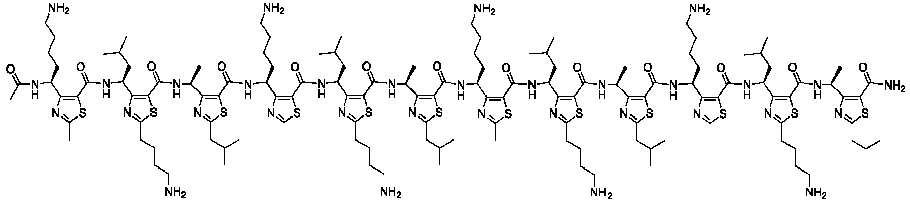

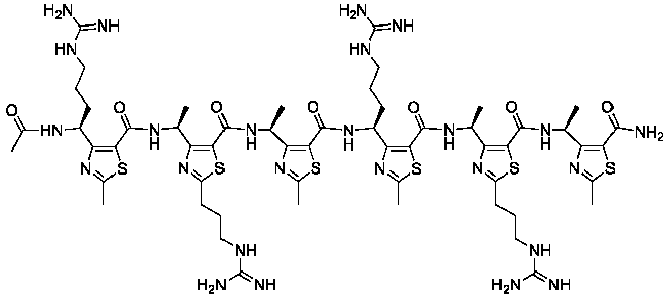

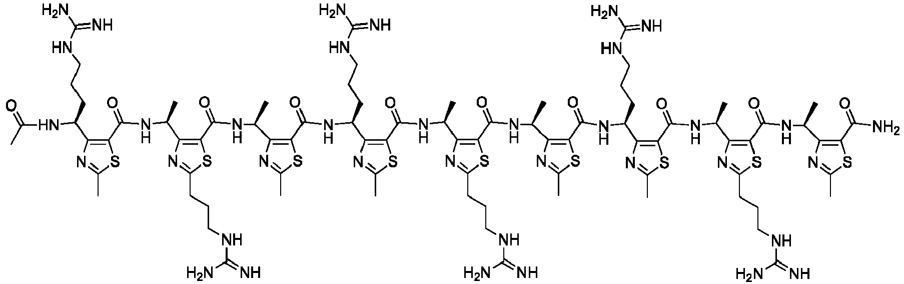

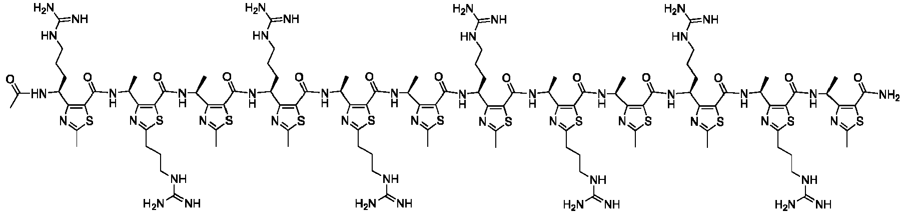

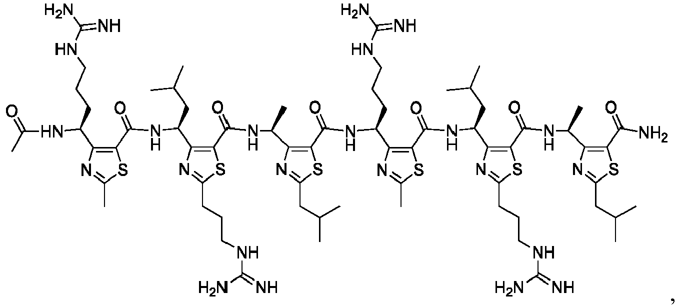

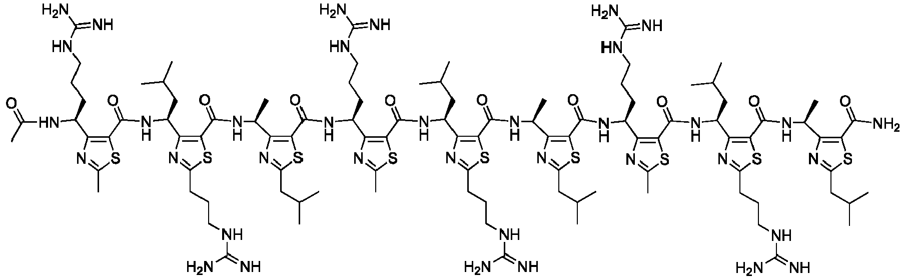

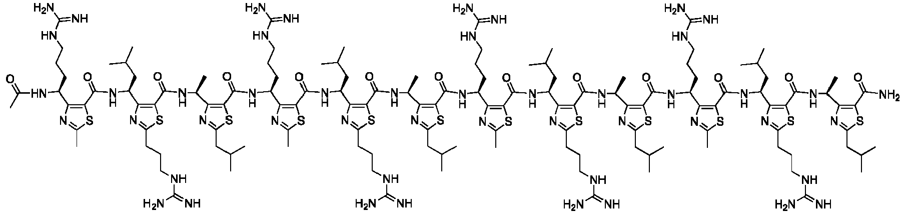

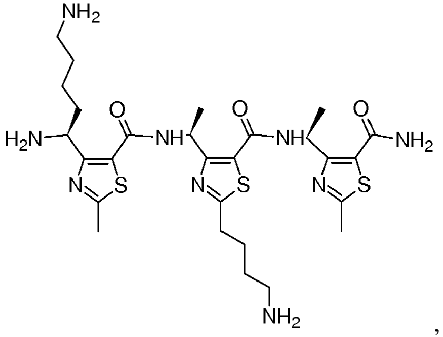

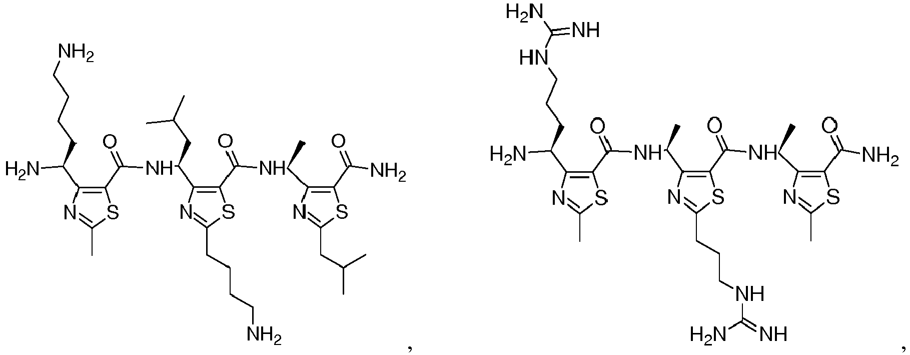

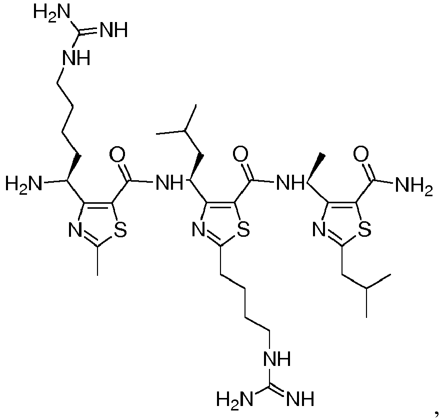

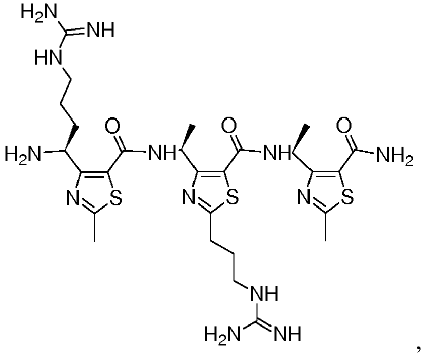

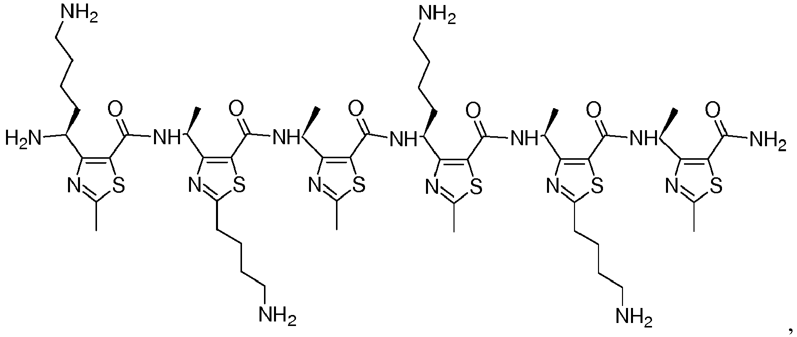

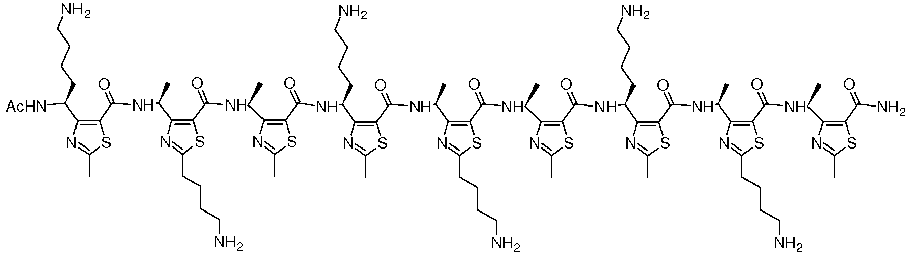

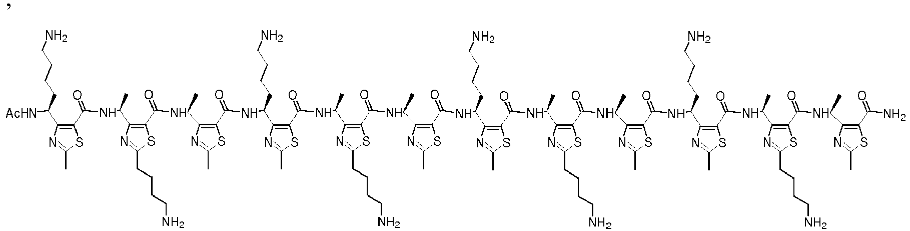

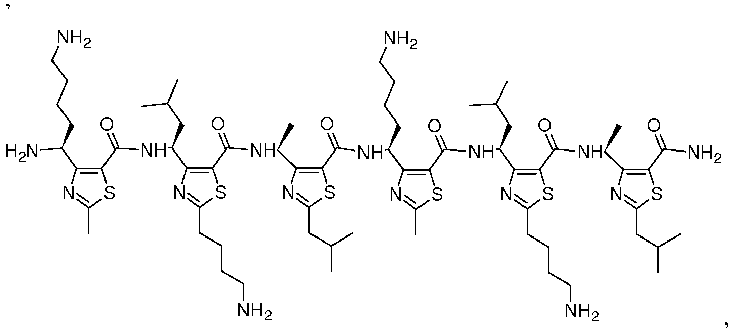

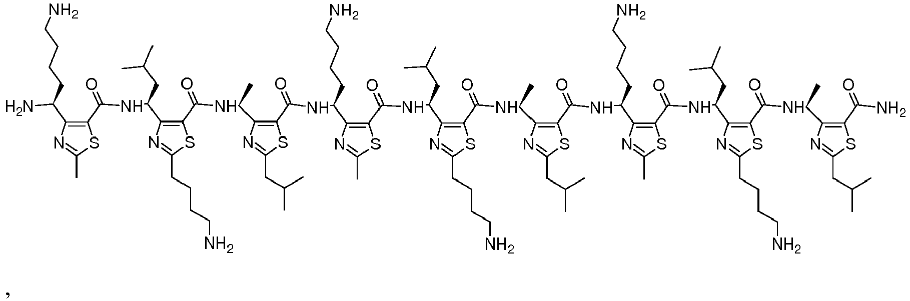

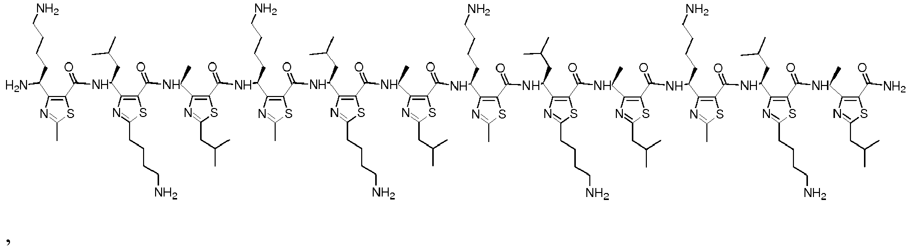

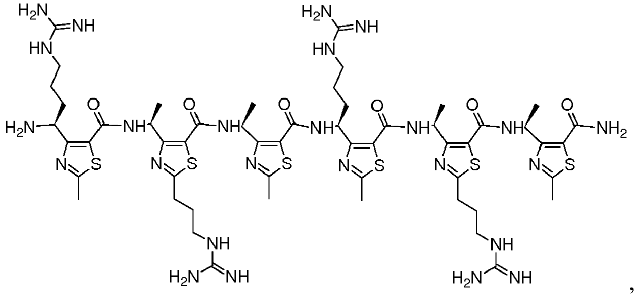

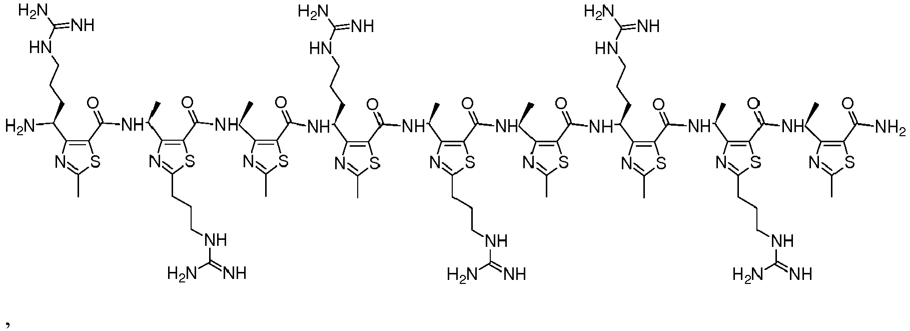

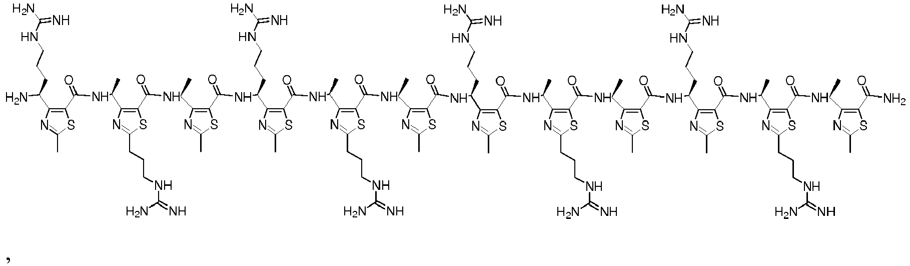

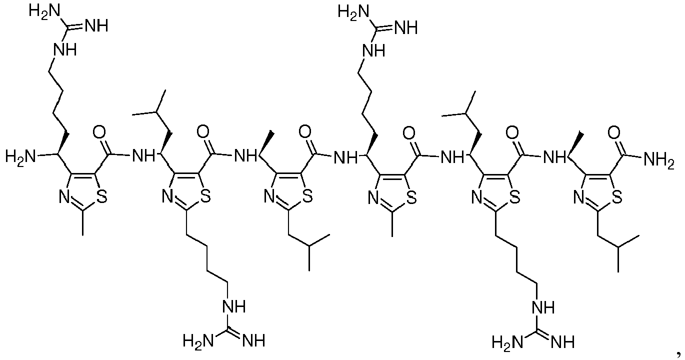

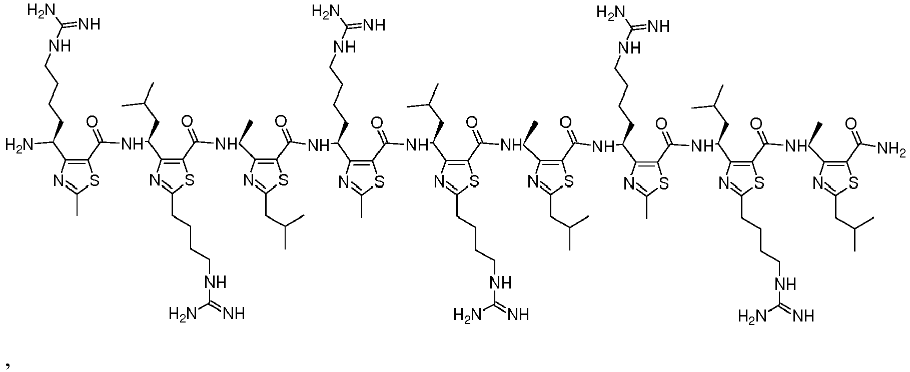

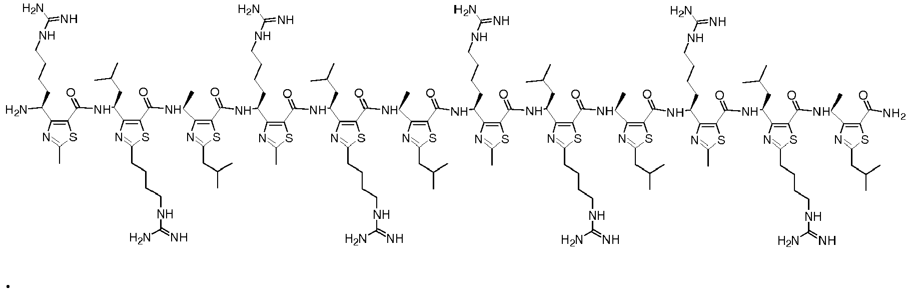

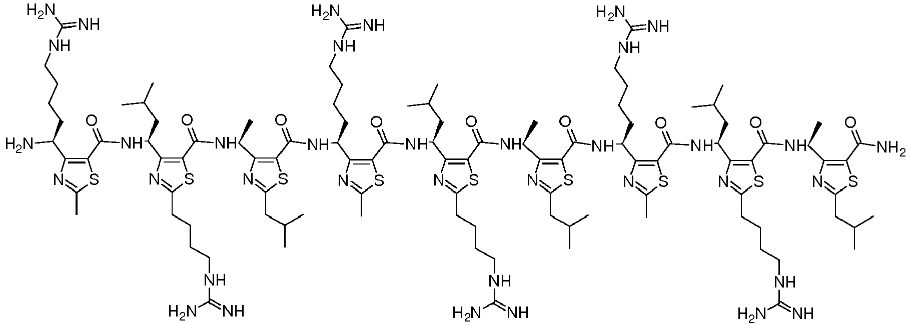

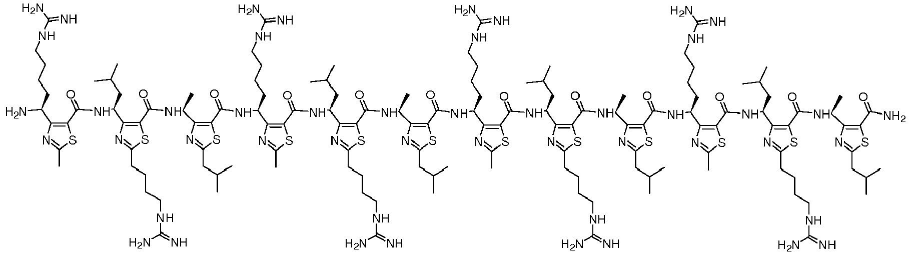

The invention also relates to a pharmaceutical composition comprising as active ingredient a compound as defined above, in association with a pharmaceutically acceptable carrier.

"Pharmaceutically-acceptable carrier" means a carrier that is useful in preparing a pharmaceutical composition or formulation that is generally safe, non-toxic, and neither biologically (except for microorganisms) nor otherwise undesirable, and includes a carrier that is acceptable for veterinary use as well as human pharmaceutical use.

Carriers are also known as excipients. The excipient employed is typically one suitable for administration to human subjects or other mammals. In making the compositions, the active ingredient is usually mixed with an excipient, diluted by an excipient, or enclosed within a carrier which can be in the form of a capsule, sachet, paper or other container. When the excipient serves as a diluent, it can be a solid, semi-solid, or liquid material, which acts as a vehicle, carrier, or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

The invention also relates to a compound as defined above for its use as a medicine.

The invention also relates to the compound as defined above, or the medicine as defined above, for its use for the treatment of infection caused by bacteria and fungi.

This means that the compound according to the invention is efficient as medicine within the context of the treatment of bacterial and fungal infections.

This disclosure also relates to the use of a compound as defined above, for the fabrication of a medicine intended for the treatment of bacterial and fungal infections.

Also, it is disclosed a method for treating a human being or an animal in a need thereof, and afflicted by an infection by bacteria or fungi, the method comprising a step of administering an efficient amount of a compound as defined above.

The medicine defined above may be associated with one or more antibiotic compound and/or an antifungi compound.

The invention also relates to the use of a compound as defined above, for the contamination of a surface infected by bacteria or fungi.

The invention also relates to Compound of formula B : 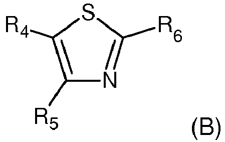 wherein R₄ is a C₁-C₄ acid, R₅ and R₆ are, independently from each other a C₁-C₅, linear or branched, alkyl, a C₁-C₅, linear or branched, alkyl, substituted by a diamine imine, protected or not, or a C₁-C₅, linear or branched, alkyl, substituted by an amine, protected or not.

The above compound corresponds to synthesis intermediates. These compounds are novel, and used for the synthesis of the above compound of formula A.

### REV12

1. Advantageously, the invention relates to the compound as mentioned above, wherein said compound is chosen from the compounds of the group consisting of : 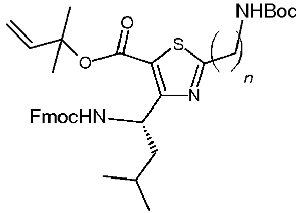 wherein n = 4, 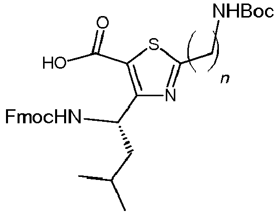 wherein n = 4, 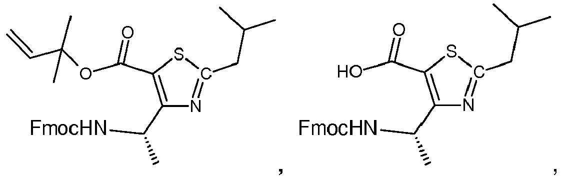 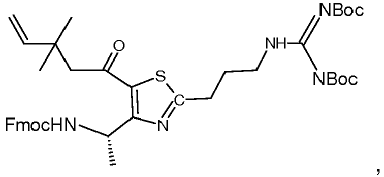 and

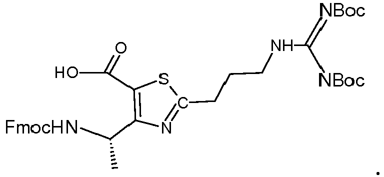

In the above compounds, Boc and Fmoc are protecting groups well known in the art and respectively corresponding tert-Butyloxycarbonyl protecting group and Fluorenylmethyloxycarbonyl protecting group.

The invention will be better explained in light of the following figures and examples.

### BRIEF DESCRIPTION OF THE DRAWINGS

[Fig.1] represents analytical HPLC analysis of oligomer 1 (Chromolith Speed Rod RP-C₁₈ 185 Pm column 50 x 4.6 mm, 5 µm with a gradient from 100% (H₂O/TFA 0.1%) to 100% (CH₃CN/TFA 0.1%) in 5 min; flow rate 4 mL/min; UV detection at 214 nm)
[Fig.2] represents analytical HPLC analysis of oligomer 2 (Chromolith Speed Rod RP-C₁₈ 185 Pm column 50 x 4.6 mm, 5 µm with a gradient from 100% (H₂O/TFA 0.1%) to 100% (CH₃CN/TFA 0.1%) in 5 min; flow rate 4 mL/min; UV detection at 214 nm)
[Fig.3] represents analytical HPLC analysis of oligomer 3 (Chromolith Speed Rod RP-C₁₈ 185 Pm column 50 x 4.6 mm, 5 µm with a gradient from 100% (H₂O/TFA 0.1%) to 100% (CH₃CN/TFA 0.1%) in 5 min; flow rate 4 mL/min; UV detection at 214 nm)
[Fig.4] represents analytical HPLC analysis of oligomer 4 (Chromolith Speed Rod RP-C₁₈ 185 Pm column 50 x 4.6 mm, 5 µm with a gradient from 100% (H₂O/TFA 0.1%) to 100% (CH₃CN/TFA 0.1%) in 5 min; flow rate 4 mL/min; UV detection at 214 nm)
[Fig.5] represents analytical HPLC analysis of oligomer 5 (Chromolith Speed Rod RP-C₁₈ 185 Pm column 50 x 4.6 mm, 5 µm with a gradient from 100% (H₂O/TFA 0.1%) to 100% (CH₃CN/TFA 0.1%) in 5 min; flow rate 4 mL/min; UV detection at 214 nm)
[Fig.6] represents analytical HPLC analysis of oligomer 6 (Chromolith Speed Rod RP-C₁₈ 185 Pm column 50 x 4.6 mm, 5 µm with a gradient from 100% (H₂O/TFA 0.1%) to 100% (CH₃CN/TFA 0.1%) in 5 min; flow rate 4 mL/min; UV detection at 214 nm)
[Fig.7] represents analytical HPLC analysis of oligomer 7 (Chromolith Speed Rod RP-C₁₈ 185 Pm column 50 x 4.6 mm, 5 µm with a gradient from 100% (H₂O/TFA 0.1%) to 100% (CH₃CN/TFA 0.1%) in 5 min; flow rate 4 mL/min; UV detection at 214 nm)
[Fig.8] represents analytical HPLC analysis of oligomer 8 (Chromolith Speed Rod RP-C₁₈ 185 Pm column 50 x 4.6 mm, 5 µm with a gradient from 100% (H₂O/TFA 0.1%) to 100% (CH₃CN/TFA 0.1%) in 5 min; flow rate 4 mL/min; UV detection at 214 nm)
[Fig.9] represents analytical HPLC analysis of oligomer 9 (Chromolith Speed Rod RP-C₁₈ 185 Pm column 50 x 4.6 mm, 5 µm with a gradient from 100% (H₂O/TFA 0.1%) to 100% (CH₃CN/TFA 0.1%) in 5 min; flow rate 4 mL/min; UV detection at 214 nm)
[Fig. 10] represents represent the description of the nomenclature and the torsion angles of the compounds.
[Fig. 11] represents the circular dichroism spectra of oligomers (3), (4) and (5) in water at 20°C.
[Fig. 12] represents the circular dichroism spectra of oligomers (6), (7), (8) and (9) in water at 20°C.
[Fig. 13] represents the FT-IR spectra (1000-2000 cm⁻¹) of oligomers (3), (4), (5), (6), (7), (8) and (9) in water at 20°C.
[Fig. 14] represents A/ Nomenclature, characteristic H-bonding network of above mentioned compounds of formula **A** and schematic representation of the position of the side chains. B/ Designed antimicrobial γ-peptides **1-9.** C/ Longitudinal and axial views of predicted conformations (*C*₉-helix) for **4, 7, 9** based on previously reported structures of ATC oligomers.
[Fig. 15] represents the syntheses of *N*-Fmoc-γ-aminoacid related as above mentioned compounds of formula **B** and γ-peptides **1-9.**
[Fig. 16] A/ Superimposition of the NH/^{γ}CH region of the DIPSY (in blue) and NOESY (in red) spectra of **3** recorded in water pH 6.5. B/ Typical Inter-residue NOEs pattern along **3.** C/ Superimposition of the 15 lowest energy NMR solution structures of **3** in water (lateral chains are omitted for clarity). D/ Representative structure of **3** (cationic lateral chains are in red). E/ FT-IR spectrum of **3** in water (pH 4.3) at 5 mM. F/ Far-UV CD spectrum of **3** in water (pH 4.3) at 25 µM between 200 and 300 nm.
[Fig. 17] represents a graphic showing the results of a time-kill study of oligomer **9** on *E. coli* over 2h incubation at 37°C. The γ-peptide **9** was evaluated at 0, 1.8 µM (MIC), 7.2 µM (4xMIC) and 14.4 µM (8xMIC).
[Fig. 18] represents electronic microscopy photos. A/, B/ and C/ SEM micrographs untreated *E. coli* on glass platelets. In isotonic medium the cells are long, intact and evenly shaped. D/, E/ and F/ After 60 min incubation with MIC of **9,** the cells appear shorter and more compact and showed protruding vesicles on their corrugated surface. G/, H/ and I/ After 60 min incubation with 8xMIC of **9,** the bacteria additionally showed small protruding bubbles on their surface leading to numerous spherical elements in the medium.
[Fig. 19] represents SEM micrographs untreated *E. coli* after 60 min incubation with 8xMIC of **9.** Left: blisters were visible on cell surface (see arrows), and right: numerous lysed cells were observed.

### EXAMPLES

### EXAMPLE 1 - SYNTHESIS

### I- General procedures

All reagents and solvents were obtained from commercial sources and used without further purification. Analytical HPLC analyses were run on an Agilent Technology 1220 Infinity LC equipped with a Chromolith Speed Rod RP-C18 185 Pm column (50 x 4.6 mm, 5 µm) with a gradient from 100% (H₂O/TFA 0.1%) to 100% (CH₃CN/TFA 0.1%) in 5 min; flow rate 4 mL/min; UV detection at 214 nm. Retention times are reported as follows: Rt = (min). LC/MS analyses were recorded on a Quattro micro™ ESI triple quadrupole mass spectrometer (Micromass, Manchester, UK) equipped with a Chromolith Speed Rod RP-C18 185 Pm column (50 x 4.6 mm, 5 µm) and an Alliance HPLC System (Waters, Milford, USA); gradient from 100% (H2O/HCO₂H 0.1%) to 100% (CH₃CN/HCO₂H 0.1%) in 3 min; flowrate 3 mL/min; UV detection at 214 nm. High-Resolution Mass Spectrometric analyses were performed with a time-of-flight (TOF) mass spectrometer fitted with an Electrospray lonisation source (ESI) in positive ion mode.

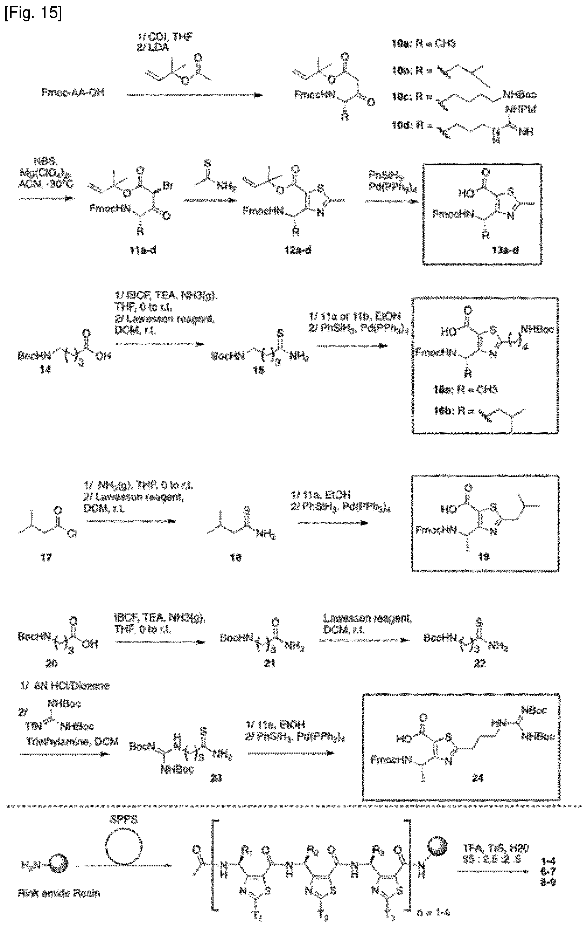

### II-Synthesis of N-Fmoc-γ-aminoacid related as above mentioned compounds of formula B - Fig. 15

### 1. Synthesis of 10a, 10b, 10c and 10d

Intermediates 10a-d, 11a-d and N-Fmoc-γ-aminoacids 13a-d, 10b, 10c and 10d have been synthesized as previously reported. (L. Mathieu, C. Bonnel, N. Masurier, L. T. Maillard, J. Martinez, V. Lisowski, Eur. J. Org. Chem. 2015, 2015, 2262-2270*).*

### 2. Synthesis of 15

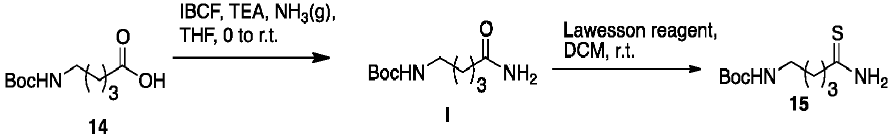

**Step 1:** To a solution of 5-(Boc-amino)pentanoic acid (1.0 equiv., 36.8 mmol, 8.0 g) in anhydrous THF at 0°C and under argon atmosphere were sequentially added IBCF (1.2 equiv.) and triethylamine (1.2 equiv.). The white suspension was stirred for 10 min until complete formation of the mixed anhydride (HPLC monitoring). Then gaseous ammonia was bubbled for 30 min at r.t. to yield *N*-Boc-aminovaleramide I as a white powder. Yield quantitative (8.02 g). ¹H NMR (400 MHz, DMSO-*d₆*) δ 1.37 (s, 9H); 1.42-1.46 (m, 4H); 2.01 (t, *J* = 7.2 Hz, 2H); 2.88 (m, 2H); 6.68 (s, 1H); 6.76 (t, 1H, *J* = 5.4 Hz); 7.21 (s, ¹H).
**Step 2:** I (1.0 equiv., 36.8 mmol, 8.02 g.) was dissolved in dry DCM (180 mL). Then Lawesson's reagent was added in one portion (0.55 equiv., 20.3 mmol, 8.19g.) and the white turbid mixture was heated at 32°C overnight. After evaporation under reduced pressure, the yellow oil was dissolved in a mixture of DCM (200 mL) and aqueous saturated NaHCO₃ solution (200 mL) which was stirred for 15 min. After transfer in a separating funnel, the organic layer was washed with brine, dried over MgSO₄, filtered and concentrated under reduced pressure to yield crude product (orange oil) which was purified by chromatography on silica gel (Et2O/Petroleum ether: 5/5 to 9/1) to yield pure N-Boc-aminopentanethioamide 15 as white solid. Yield 50 % (4.28 g). ¹H NMR (400 MHz, DMSO-*d₆*) δ 1.37 (s, 11H); 1.60 (quin, *J* = 7.5 Hz, 2H); 2.44 (t, *J* = 7.5 Hz, 2H); 2.89 (m, 2H); 6.78 (t, *J* = 5.4 Hz, 1H); 9.11 (s, 1H); 9.31 (s, 1H). LC Rt = 1.36 min. LC-MS: (ESI+): m/z 255.1 ([M+Na]⁺) 90%, 233.1 ([M+H]⁺) 25%, 133.1 ([M-Boc+H]⁺) 100%.

### 3. Synthesis of 16a

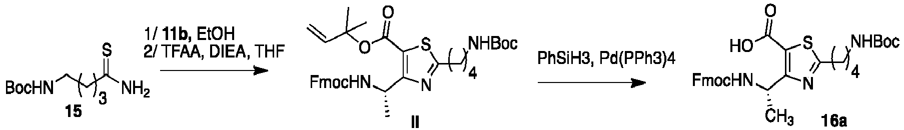

A solution of 11a (1.0 equiv.) and 15 (2.0 equiv.) in EtOH was stirred 90 min at r.t. then 30 min at 40°C. After evaporation of the solvent under reduced pressure, the crude was purified by flash chromatography (Cyclohexane/EtOAc: 90/10 to 60/40) to yield **II** as a clear oil. Yield 56% (697 mg). ¹H NMR (400 MHz, CDCl₃) δ 1.44 (s, 9H); 1.47 (d, *J* = 6.7 Hz, 3H); 1.59 (m, 2H); 1.63 (s, 3H); 1.65 (s, 3H); 1.82 (quin, *J* = 7.6 Hz, 2H), 2.98 (t, *J* = 7.6 Hz, 2H); 3.16 (brm, 2H); 4.22 (t, *J* = 7.1 Hz, 1H); 4.35 (d, *J* = 7.8 Hz, 2H); 4.66 (br, 1H); 5.15 (d, *J* = 11.1 Hz, 1H); 5.26 (d, *J* = 17.8 Hz, 1H); 5.67 (m, 1H); 6.02 (brm, 1H); 6.16 (dd, *J* = 11.1, 17.8 Hz, 1H); 7.30 (m, 2H); 7.39 (t, *J* = 7.4 Hz, 2H); 7.61 (t, *J* = 7.7 Hz, 2H); 7.76 (d, *J* = 7.4 Hz, 2H). ¹³C NMR (100 MHz, CDCl₃) δ 22.1; 26.7 (2C); 27.9; 28.6 (3C); 29.5; 33.2; 40.2; 46.7; 47.4; 66.9; 79.4; 83.2; 113.6; 120.1 (2C); 122.8; 125.3 (2C); 127.1 (2C); 127.8 (2C); 141.4 (2C); 142.0; 144.1; 144.3; 155.7; 156.1; 160.4; 162.6; 174.5. LC Rt = 2.62 min. LC-MS: (ESI+): m/z 656.1 ([M+Na]⁺) 25%, 634.1 ([M+H]⁺) 100%, 578.0 ([M-*t*Bu+2H]⁺) 30%, 534.1 ([M-Boc+H]⁺) 80%, 466.0 ([M-Boc-C₅H₉+H]⁺) 55%. HRMS (ESI+) m/z calcd. for [C₃₅H₄₄N₃O₆S]⁺: [M+H]⁺ 634.2949, found 634.2951.

The O-dimethylallylester II (1.10 mmol) was dissolved in dry THF (5 mL). Tetrakis (triphenylphosphine)palladium(0) (3 mol %) was then added to the solution and the orange mixture was stirred for 5 min at r.t.. Then PhSiH₃ (1.2 equiv.) was added and the solution was stirred for 30 min (HPLC monitoring). The solvent was then evaporated under vacuum to yield **16a** as a brown foam (630 mg).). LC Rt = 2.12 min. LC-MS: (ESI+): m/z 588.2 ([M+Na]⁺ 20%, 566.2 ([M+H]⁺) 30%, 466.2 ([M-Boc+H]⁺) 100%.

### 4. Synthesis of 16b

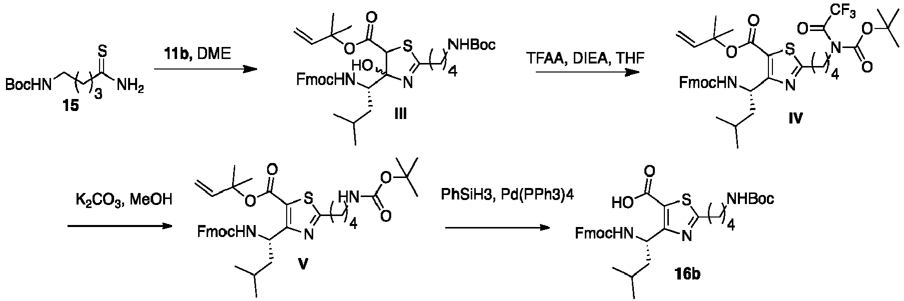

**Step 1: 15** (1.1 equiv., 5.41 mmol) was dissolved in DME (35 mL) and then KHCO3 (8.0 equiv., 39.3 mmol) was added in one portion. The mixture was vigorously stirred for 30 min at r.t.. 11b was dissolved in 40 mL of DME and added to the mixture with a dropping funnel at 0°C. The reaction was stirred overnight at r.t.. The crude was filtered to remove unsoluble KHCO₃ and the clear filtrate was evaporated under reduced pressure. The resulting yellow oil was dissolved in EtOAc (100 mL) then washed with water (1 × 100 mL) and brine (1 × 50 mL), dried over MgSO4 and filtered. The solvent was evaporated to yield the corresponding thiazoline III as an orange foam. Yield quantitative (3.44 g). LC Rt = 2.57 min. LC-MS: (ESI+): m/z 694.3 ([M+H]⁺) 100%.
**Step 2:** The thiazoline III was dissolved in 50 mL of dry THF at -15°C. Diisoproylethylamine (8.0 equiv., 39.3 mmol, 6.80 mL) was added and the mixture was stirred for 15 min. Then trifluoroacetic anhydride (4.0 equiv., 19.6 mmol, 2.74 mL) was added dropwise (4-5 min). After 30 min of stirring at -15°C, a second equiv. of trifluoroacetic anhydride was added to the orange-red solution in order to consume remaining thiazoline. After 10 min of stirring at -15°C the mixture was diluted with DCM (100 mL) and washed with aqueous saturated NaHCO₃ solution (2 × 100 mL), then with aqueous saturated KHSO₄ solution (1 × 100 mL) and brine (1 × 50 mL). The organic layer was dried over MgSO₄, filtered and concentrated under reduced pressure to yield 4.19 g of -15°C IV as a red foam.
**Step 3:** KHCO₃ (2.0 equiv., 9.82 mmol, 983 mg) was added to a solution of **IV** dissolved in dry MeOH (50 mL). The suspension was stirred overnight at r.t. then diluted with DCM (80 mL) and washed with aqueous saturated NaHCO₃ solution (1 × 50 mL) and brine (2 × 70 mL). The organic layer was dried over MgSO₄, filtered, concentrated under reduced pressure and filtered through a Celite pad to yield crude **V** as an orange foam which was used for the next step without further purification. Yield 93% (3.09 g). ¹H NMR (400 MHz, CDCl₃) δ 0.95 (d, *J* = 6.4 Hz, 3H); 0.97 (d, *J* = 6.4 Hz, 3H); 1.44 (s, 9H); 1.55-1.62 (m, 3H); 1.66 (s, 7H); 1.70-1.83 (m, 3H); 2.96 (t, *J* = 7.6 Hz, 2H); 3.15 (brm, 2H); 4.21 (t, *J* = 7.0 Hz, 1H); 4.30 (dd, *J* = 7.5, 10.5 Hz, 1H); 4.39 (dd, *J* = 7.5, 10.5 Hz 1H); 4.67 (brm, 1H); 5.14 (d, *J* = 11.0 Hz, 1H); 5.26 (d, *J* = 17.5 Hz, 1H); 5.70 (m, 1H); 5.84 (brm, 1H); 6.19 (dd, *J* = 11.0, 17.5 Hz, 1H); 7.29 (m, 2H); 7.38 (t, *J* = 7.3 Hz, 2H); 7.58 (d, *J* = 7.1 Hz, 1H); 7.59 (d, *J* = 5.3 Hz, 1H) 7.75 (d, *J* = 7.3 Hz, 2H). ¹³C NMR (100 MHz, CDCl₃) δ 22.3; 23.3; 25.1; 26.7; 26.8; 27.1; 28.5 (3C); 29.5; 33.2; 40.2; 45.2; 47.4; 49.0; 66.7; 79.4; 83.3; 113.5; 120.1 (2C); 123.4; 125.3 (2C); 127.1 (2C); 127.7 (2C); 141.4 (2C); 142.1; 144.1; 144.2; 155.9; 156.1; 160.5; 162.3; 174.4. LC Rt = 2.92 min. LC-MS: (ESI+): m/z 698.2 ([M+Na]⁺) 55%, 676.2 ([M+H]⁺) 100%, 576.1 ([M-Boc+2H]⁺) 75%, 508.1 ([M-C₅H₉+2H]⁺), 50%. HRMS (ESI+) m/z calcd. for [C₃₈H₅₀N₃O₆S]⁺: [M+H]⁺ 676.3421, found 676.3420.

The O-dimethylallylester V (4.5 mmol) was dissolved in dry THF (25 mL). Tetrakis (triphenylphosphine)palladium(0) (3 mol %) was then added to the solution and the orange mixture was stirred for 5 min at r.t.. Then PhSiH₃ (1.2 equiv.) was added and the solution was stirred for 30 min (HPLC monitoring). The solvent was then evaporated under vacuum to yield **16b** as a brown foam (1.25 g). LC Rt = 2.34 min. LC-MS: (ESI+): m/z 630.2 ([M+Na]⁺) 5%, 608.2 ([M+H]⁺) 25%; 552.1 ([M-*t*Bu+2H]⁺) 15%; 508.1 ([M-Boc+2H]⁺) 100%.

### 5. Synthesis of 19

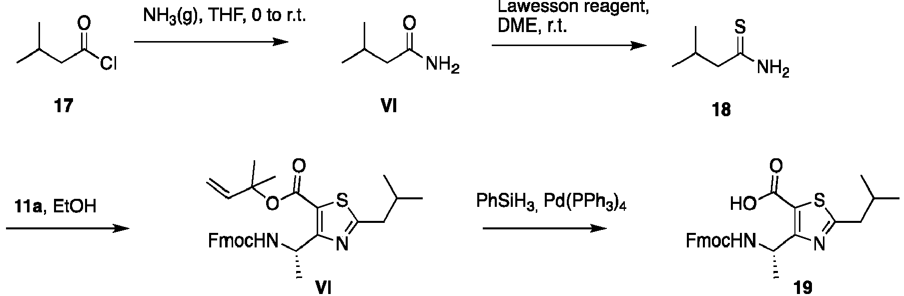

**Step 1:** Isovaleryl chloride (1.0 equiv., 16.6 mmol, 2.0 mL) was diluted with 20 mL of dry THF under an argon atmosphere. Then ammoniac was bubbled into the solution for 30 min at 0°C and the white turbid mixture was evaporated to yield quantitatively **VI** as a white powder (2.84 g). ¹H NMR (400 MHz, DMSO-*d*₆) δ 0.83 (d, *J* = 6.4 Hz, 6H); 1.90-1.93 (m, 3H); 6.72 (s, 1H); 7.21 (s, 1H).
**Step 2: VI** (1.0 equiv., 16.6 mmol, 2.8 g) was dissolved in dimethoxyethane (DME, 120 mL) and Lawesson's reagent (0.55 equiv., 9.1 mmol, 3.7 g) was then added in one portion. The mixture was refluxed at 100°C for 2 h (HPLC monitoring). Solvent was evaporated and the yellowish residue was dissolved in a mixture of EtOAc (100 mL) and aqueous saturated NaHCO₃ solution (100 mL) which was stirred for 15 min. After transfer in a separating funnel, the organic layer was washed with brine, dried over MgSO₄, filtered and concentrated under reduced pressure to yield crude product (yellow oil) which was purified by flash chromatography on silica gel (DCM 100% to DCM/MeOH: 94/6) to yield pure **18** as slight yellow crystals. Yield 43 % (841 mg). ¹H NMR (400 MHz, DMSO-*d*₆) δ 0.98 (d, *J* = 6.6 Hz, 6H); 2.23 (non, *J* = 6.9 Hz, 1H); 2.50 (d, 2H, *J* = 6.9 Hz); 6.90 (br, 1H); 7.76 (br, 1H). LC Rt = 1.00 min. LC-MS: (ESI+): m/z 118.2 ([M+H]⁺) 100%.
**Step 3:** A solution of **11a** (1.0 equiv., 4.5 mmol, 2.25 g) and **18** (1.6 equiv.) in EtOH was stirred 4 h at 40°C. After evaporation of the solvent, the crude product was purified by flash chromatography (Cyclohexane 100% to Cyclohexane/EtOAc: 80/20) to yield pure **VI** as a clear oil. Yield 41% (952 mg). ¹H NMR (400 MHz, CDCl₃) δ 0.99 (s, 3H); 1.01 (s, 3H); 1.48 (d, *J* = 6.7 Hz, 3H); 1.65 (s, 6H); 2.11 (non, *J* = 6.7 Hz, 1H); 2.82 (d, *J* = 7.1 Hz, 2H); 4.23 (t, *J* = 7.00 Hz, 1H); 4.35 (m, 2H); 5.15 (d, *J* = 10.9 Hz, 1H); 5.27 (d, *J* = 17.3 Hz, 1H) 5.68 (m, 1H); 6.02 (d, *J* = 8.7 Hz, 1H); 6.18 (dd, *J* = 10.9, 17.3 Hz, 1H); 7.29 (d, *J* = 7.6 Hz, 1H); 7.31 (d, *J* = 7.3 Hz, 1H); 7.39 (d, *J* = 7.6 Hz, 1H); 7.41 (d, *J* = 7.3 Hz, 1H); 7.59 (d, *J* = 4.9 Hz, 1H); 7.61 (d, *J* = 7.3 Hz, 1H); 7.76 (d, *J* = 7.6 Hz, 2H). ¹³C NMR (100 MHz, CDCl₃) δ 22.3; 22.4 (2C); 26.7 (2C); 29.9; 42.6; 46.7; 47.4; 66.8; 83.1; 113.5; 120.1 (2C); 122.7; 125.3 (2C); 127.1 (2C); 127.7 (2C); 141.4 (2C); 142.0; 144.2; 144.3; 155.7; 160.5; 162.5; 174.0. LC Rt = 2.85 min. LC-MS: (ESI+): m/z 541.1 ([M+Na]⁺) 40%, 519.1 ([M+H]⁺) 85%, 451.1 ([M-C₅H₉+2H]⁺) 100%. HRMS (ESI+) m/z calcd. for [C₃₀H₃₅N₂O₄S]⁺: [M+H]⁺ 519.2318, found 519.2318.

The *O*-dimethylallylester **VI** (1.8 mmol) was dissolved in dry THF (10 mL). Tetrakis (triphenylphosphine)palladium(0) (3 mol %) was then added to the solution and the orange mixture was stirred for 5 min at r.t.. Then PhSiH₃ (1.2 equiv.) was added and the solution was stirred for 30 min (HPLC monitoring). The solvent was then evaporated under vacuum to yield **18** as a brown foam (1.25 g).). LC Rt = 2.23 min. LC-MS: (ESI+): m/z 451.2 ([M+H]⁺) 100%.

### 6. Synthesis of 24

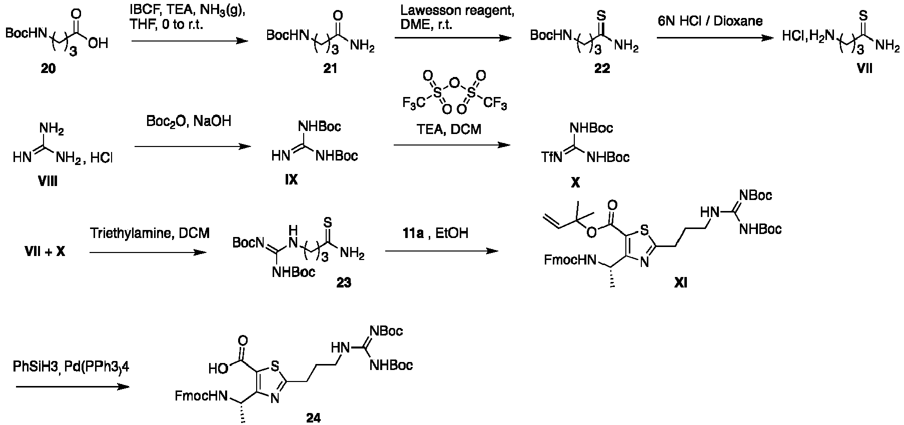

**Step 1 - synthesis of 21:** To a solution of 4-(Boc-amino)butanoic acid **20** (1.0 equiv., 60 mmol, 12.2 g) in anhydrous THF at 0°C and under argon atmosphere were sequentially added IBCF (1.2 equiv.) and triethylamine (1.2 equiv.). The white suspension was stirred for 10 min until complete formation of the mixed anhydride (HPLC monitoring). Then gaseous ammonia was bubbled for 30 min at r.t. to yield 4-(Boc-amino)-butylamide 22 as a white powder. Yield 92% (11.14 g). ¹H NMR (400 MHz, CDCl₃) δ 1.43 (s, 9H); 1.79 (quin, *J* = 7.1 Hz, 2H); 2.26 (t, *J* = 7.1 Hz, 1H); 2.36 (t, *J* = 7.1 Hz, 1H); 3.17 (brm, H); 3H missing.
**Step 2 -synthesis of 22: 21** (1.0 equiv., 55.1 mmol, 11.1 g) was dissolved in dimethoxyethane (DME, 230 mL) and Lawesson's reagent (0.55 equiv., 30.3 mmol, 12.25 g) was then added in one portion. The mixture was refluxed at 100°C for 2 h (HPLC monitoring). Solvent was evaporated and the yellowish residue was dissolved in a mixture of DCM (100 mL) and aqueous saturated NaHCO₃ solution (100 mL) which was stirred for 15 min. After transfer in a separating funnel, the organic layer was washed with brine, dried over MgSO₄, filtered and concentrated under reduced pressure to yield crude product (yellow oil) which was filtered on a Celite pad prior to purification by chromatography on silica gel (Cyclohexane/EtOAc: 60/40 to 30/70) to yield pure **22** as slight yellow crystals. Yield 38 % (4.94 g). ¹H NMR (400 MHz, CDCl₃) δ 1.43 (s, 9H); 1.60 (quin, *J* = 6.2 Hz, 2H); 2.72 (t, *J* = 6.2 Hz, 2H); 3.21 (q, *J* = 6.4 Hz, 2H); 4.79 (br, 1H); 7.64 (br, 1H); 8.71 (br, 1H). LC Rt = 1.24 min. LC-MS: (ESI+): m/z 241.1 ([M+Na]⁺) 5%, 219.1 ([M+H]⁺) 20%, 163.1 ([M-*t*Bu+2H]⁺) 100%, 119.1 ([M-Boc+2H]⁺) 45%.
**Step 3** - **synthesis of VII:** A solution of 6N HCl in 1,4-dioxane (50 mL) was added to **22** (1.0 equiv., 22.4 mmol, 4.89 g) and the mixture was stirred for 1 h at r.t. The reaction was monitored by HPLC. The solvent was evaporated *in vacuo.* Co-evaporation with MeOH yielded **VII** as a yellow oil. Quantitative yield (3.76 g). LC Rt = 0.30 min (≥ 95%). LC-MS: (ESI+): m/z 118.9 ([M+H]⁺) 100%.
**Step 4** - **synthesis of IX:** 1,4-dioxane (80 mL) was added to a solution of guanidine hydrochloride (1.0 equiv., 40 mmol, 3.82 g) and sodium hydroxide (4.0 equiv., 160 mmol, 6.42 g) dissolved in water (40 mL). The resulting mixture was cooled to 0°C. Di-*tert*-butyl-dicarbonate (2.2 equiv., 88 mmol, 19.2 g) was added in one portion and the reaction mixture was allowed to warm to room temperature overnight. Mixture was concentrated *in vacuo* to 1/3 of its original volume. The resulting suspension was diluted with water (80 mL) and extracted with EtOAc (3 × 90 mL). The combined organic layers were washed with 10% citric acid (150 mL), water (150 mL) and brine (150 mL) and dried with magnesium sulfate. After filtering and removal of the solvent under reduced pressure, the crude was purified by chromatography on silica gel (DCM/MeOH 99/1 to 96/4) to yield **IX** as a white powder. Yield 53% (5.46 g). ¹H NMR (400 MHz, CDCl₃) δ 1.41 (s, 18H); 8.48 (br, 1H); 10.35 (br, 2H). LC Rt = 1.48 min (≥ 95%). LC-MS: (ESI+): m/z 260.1 ([M+H]⁺) 70%, 204.3 ([M-*t*Bu+2H]⁺) 90%, 147.6 ([M-2*t*Bu+3H]⁺) 100%. LC Rt = 2.26 min (≤ 5%; *N,N',N"*-Tri(Boc)-guanidine). LC-MS: (ESI+): m/z 360.1 ([M+H]⁺) 50%, 304.3 ([M-*t*Bu+2H]⁺) 75%, 248.1 ([M-2*t*Bu+3H]⁺) 75%, 192.0 ([M-3*t*Bu+4H]⁺) 100%, 148.1 ([M-2*t*Bu-Boc+4H]⁺) 60%.
**Step 5** - **synthesis of X:** A solution of **IX** (1.0 equiv., 21 mmol, 5.44 g) and triethylamine (1.08 equiv., 22.7 mmol, 3.1 mL) in anhydrous DCM (105 mL) was cooled to -78°C under an argon atmosphere. Triflic anhydride (1.05 equiv., 22.0 mmol, 3.71 mL) was added dropwise at a rate such that reaction temperature does not exceed -70°C. After the addition was completed, the reaction mixture was allowed to warm to room temperature within 4 hours. The solution was transferred to a separation funnel, washed with 2N KHSO₄ and water and dried over magnesium sulfate. The organic layer was dried over MgSO₄, filtered and concentrated under reduced to yield crude **X** as a slight yellow powder which was used for the next step without further purification. Yield 89% (7.3 g). ¹H NMR (400 MHz, CDCl₃) δ 1.46 (s, 18H); 11.05 (br, 2H). LC Rt = 2.26 min (≥ 95%). LC-MS: (ESI+): m/z 392.0 ([M+H]⁺) 5%, 336.1 90%, 280.1 95%, 236.2 100%.
**Step 6-synthesis of 23: VII** (1.1 equiv., 20.35 mmol, 3.15g) was dissolved in a 1:1 mixture of DCM: MeOH (80mL) and added in one time to a solution of **X** (1.0 equiv., 18.5 mmol, 7.24 g) and triethylamine (1.0 equiv., 18.5 mmol, 2.53 mL) dissolved in DCM (80 mL). After 80 min of stirring at r.t., reaction was stopped. Mixture was diluted with DCM (60 mL) and washed with water (100 mL), KHSO₄ (100 mL), NaHCO₃ (100 mL) and brine (100 mL). After filtering and removal of the solvent under reduced pressure, crude was purified by flash chromatography on silica gel (Cyclohexane/EtOAc 90/10 to 65/35) to yield pure **23** as a white powder. Yield 47% (3.14 g). ¹H NMR (400 MHz, CDCl₃) δ 1.44 (s, 9H); 1.49 (s, 9H); 1.96 (m, 2H); 2.81 (m, 2H); 3.44 (q, *J* = 6.1 Hz, 2H); 7.72 (br, 1H); 8.58 (t, *J* = Hz, 1H); 10.41 (br, 1H); 11.36 (br, 1H). ¹³C NMR (100 MHz, CDCl₃) δ 28.1 (3C); 28.3 (3C); 29.4; 39.0; 43.4; 79.9; 83.9; 153.2; 157.3; 162.5; 209.4. LC Rt = 1.65 min. LC-MS: (ESI+): m/z 361.0 ([M+H]⁺) 100%, 305.1 ([M-*t*Bu+2H]⁺) 100%, 249.1 ([M-2*t*Bu+3H]⁺) 65%, 205.1 ([M-2*t*Bu-Boc+4H]⁺) 30%. HRMS (ESI+) m/z calcd. for [C₁₅H₂₉N₄O₄S]⁺: [M+H]⁺ 361.1910, found 361.1910.
**Step 7** - **synthesis of XI:** A solution of **11a** (1.0 equiv., 4.91 mmol, 2.25 g) and **23** (1.5 equiv.) in EtOH was stirred 90 min at r.t. then 30 min at 40°C. After evaporation of the solvent, the crude was purified by flash chromatography (DCM 100% to DCM/EtOAc: 80/20) to yield **XI** as a neon yellow foam. Yield 27% (1.03 g). ¹H NMR (400 MHz, CDCl₃) δ 1.48 (brm, 3H); 1.50 (s, 18H); 1.63 (m, 6H); 2.08 (t, *J* = 7.2 Hz, 1H); 3.03 (t, *J* = 7.6 Hz, 2H); 3.53 (brm, 2H); 4.22 (t, *J* = 7.1 Hz, 1H); 4.33 (d, *J* = 7.1 Hz, 2H); 5.15 (d, *J* = 10.9 Hz, 1H); 5.24 (d, J = 17.4 Hz, 1H); 5.26 (d, *J* = 17.4 Hz, 1H) 5.69 (m, 1H); 6.02 (d, *J* = 9.3 Hz, 1H); 6.16 (m, 1H, H₂); 7.29 (td, *J* = 7.4, 2.2 Hz, 2H); 7.38 (t, *J* = 7.5 Hz, 2H); 7.59 (d, *J* = 6.7 Hz, 1H); 7.61 (d, *J* = 6.7 Hz, 1H); 7.75 (d, *J* = 7.5 Hz, 2H). ¹³C NMR (100 MHz, CDCl₃) δ 22.2; 26.7 (2C); 28.2 (3C); 28.5 (3C); 29.0; 30.8; 39.8; 46.7; 47.4; 66.9; 79.5; 83.2; 83.4; 113.6; 120.1 (2C); 122.9; 125.3 (2C); 127.1 (2C); 127.7 (2C); 141.4 (2C); 142.0; 144.1; 144.3 (C₁₄); 153.4; 155.7; 156.4; 160.3; 162.8; 163.8; 173.4. LC Rt = 2.58 min. LC-MS: (ESI+): m/z 762.4 ([M+H]⁺) 100%, 662.3 ([M-Boc+2H]⁺) 75%, 562.2 ([M-2Boc+3H]+) 20%. HRMS (ESI+) m/z calcd. for [C₄₀H5₂N₅O₈S]⁺: [M+H]⁺ 762.3543, found 762.3537.
**Step 8** - synthesis of 24:
   The O-dimethylallylester **XI** (1.3 mmol) was dissolved in dry THF (5 mL). Tetrakis (triphenylphosphine)palladium(0) (3 mol %) was then added to the solution and the orange mixture was stirred for 5 min at r.t.. Then PhSiH₃ (1.2 equiv.) was added and the solution was stirred for 30 min (HPLC monitoring). The solvent was then evaporated under vacuum to yield **24** as a brown foam (1.25 g). LC Rt = 2.18 min. LC-MS: (ESI+): m/z 694.2 ([M+H]⁺) 100%, 594.2 ([M-Boc+2H]⁺) 40%, 494.2 ([M-2Boc+3H]⁺) 5%.

### III. Synthesis of sequences 1, 2, 3, 4, 6, 7, 8, 9 related as above mentioned compounds of formula A

### 1. General procedure of solid phase synthesis

Solid-phase synthesis was performed on a ChemMatrix® Rink Amide resin loaded at 0.49 mmol/g using Fmoc/*t*-Bu chemistry. Resin was swollen in *N-*methylpyrrolidone (NMP) for 5-10 minutes and filtered. *N*-Fmoc-γ-aminoacid (1.5 equiv.), DIC (1.5 equiv.), Oxyma Pure (1.5 equiv.), and NMP were added in this order for each peptide coupling (overnight at r.t.). Resin was washed using the following procedure: 3 × DMF, 3 × DCM, 3 × MeOH, 1 × DMF, 1 × MeOH, 1 × DCM. Each coupling was followed by a capping step with a Ac₂O/DCM 1/1 vv solution (1 × 5 min at r.t.) and washed. Deprotection at the *N*-terminus was performed using a 20% piperidine/DMF solution (3 × 10 min at r.t.) and the resin was then washed before the next coupling. Deprotection and coupling steps were monitored by Kaiser test. After completion of the oligomerization process, the foldamer was *N*-deprotected (3 × 10 min at r.t.), capped (2 × 5 min at r.t.) and cleaved from the resin with a TFA/TIS/H₂O 95/2.5/2.5 vvv solution (2 × 90 min at r.t.). The resin was washed (1 × DCM and 1 × cleavage cocktail) and filtered to retrieve the filtrate which was evaporated under reduced pressure. Crude foldamer was precipitated with cold diethylether, centrifugated 10 min at 5°C and lyophilized. Purification by preparative RP-HPLC was performed on a Waters system controller equipped with a C₁₈ Waters Delta-Pack column (100 × 40 mm, 100 Å) flow 50 mL/min; UV detection at 214 nm using a Waters 486 Tunable Absorbance Detector and a linear gradient of A = H₂O (0.1 % TFA) and B = CH₃CN (0.1 % TFA).

### 2. Oligomer 1

**1** was synthesized according to solid phase general procedures starting from 1 mmol of resin. Crude (≈ 0.25-0.3 mmol) was purified by preparative RP-HPLC to yield the pure peptide as a pulverulent white powder. Yield 16 % (40 mg). Gradient (% of eluent B): 0→10 (2 min), 10→15 (3 min), 15→40 (25 min). LC Rt = 1.91 min. LC-MS: (ESI+): m/z 339.6 ([M+2H]²⁺) 100%.

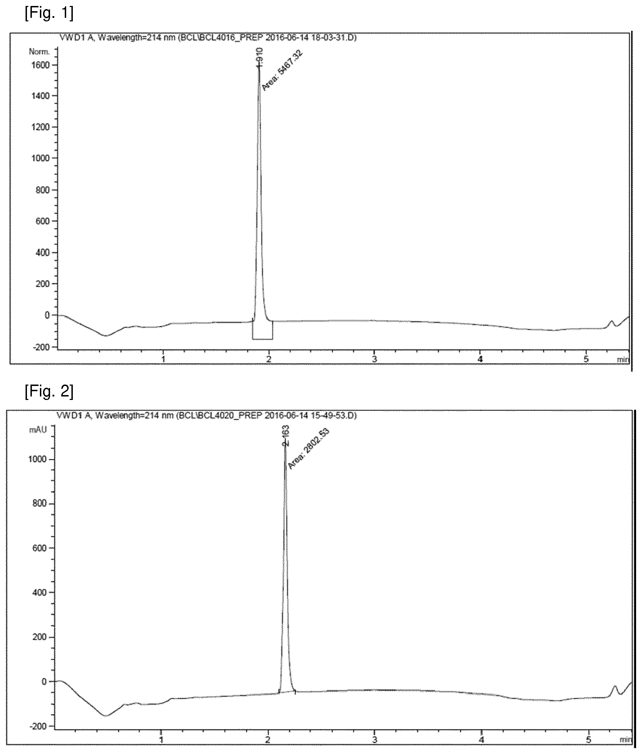

### Results of the RP-HPLC are shown in Fig. 1.

### 3. Oligomer 2

**2** was synthesized according to solid phase general procedures described in Section *VI.1.* starting from 0.7 mmol of the corresponding resin-linked fully protected trimer. Crude (≈ 0.20-0.30 mmol) was purified by preparative RP-HPLC to yield the pure peptide as a pulverulent white powder. Yield 12% (45 mg). Gradient (% of eluent B): 0→15 (2 min), 15→20 (3 min), 20→40 (20 min). LC Rt = 2.16 min. LC-MS: (ESI+): m/z 649.5 ([M+2H]²⁺) 10%, 433.1 ([M+3H]³⁺) 60%, 325.3 ([M+4H]⁴⁺) 100%.

### Results of the RP-HPLC are shown in Fig. 2.

### 4. Oligomer 3

**3** was synthesized according to solid phase general procedures described in Section *VI.1.* starting from 0.4 mmol of the corresponding resin-linked fully protected hexamer. Crude (≈ 0.20-0.25 mmol) was purified by preparative RP-HPLC to yield the pure peptide as a pulverulent white powder. Yield 16% (61 mg). Gradient (% of eluent B): 0→15 (2 min), 15→23 (5 min), 23→40 (17 min). LC Rt = 2.26 min. LC-MS: (ESI+): m/z 958.5 ([M+2H]²⁺) 10%, 639.4 ([M+3H]³⁺) 35%, 325.3 ([M+4H]⁴⁺) 75%, 384.0 ([M+5H]⁵⁺) 100%, 320.2 ([M+6H]⁶⁺) 15%.

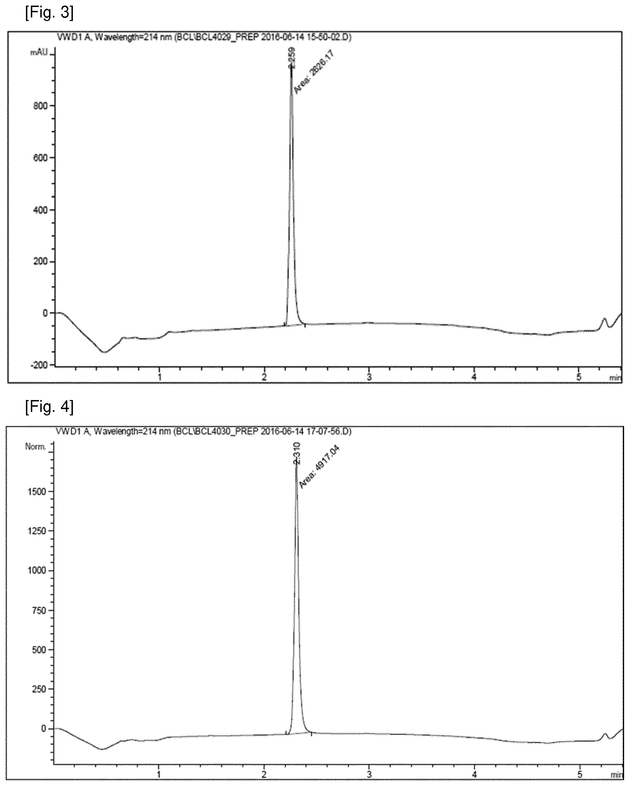

### Results of the RP-HPLC are shown in Fig. 3.

The following table 1 represents the ¹HNMR chemical shifts for **3** in H2O/D2O (9:1) pH 6.5 at 283 K. The nomenclature of 3 is described Fig 10. **3** has the following formula :

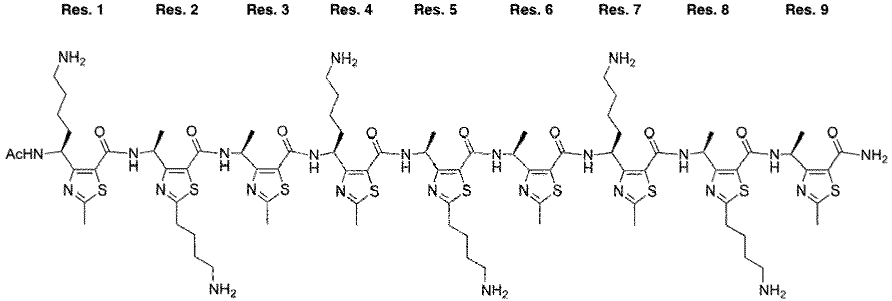

**[Table 1]**

| ***Residue*** | ***HN*** | ***^{γ}CH*** | ***^{δ}CH*** | ***Others*** |
|---|---|---|---|---|
| **Ac** | - | - | - | CH₃ 2.07 |
| **Res. 1** | 8.78 | 5.08 | 1.89 | ^{ε}CH₂ 0.89 ^{ζ}CH₂ 1.44 ^{η}CH₂ 2.76 ^{τ1}CH₃ 2.69 |
| **Res. 2** | 10.13 | 5.46 | 1.56 | ^{τ1}CH₂ 3.08 ^{τ2}CH₂ 1.89 ^{τ3}CH₂ 1.77 ^{τ4}CH 2.97 |
| **Res. 3** | 9.91 | 5.52 | 1.57 | ^{τ1}CH₃ 2.73 |
| **Res. 4** | 9.70 | 5.37 | 2.05 | ^{ε}CH₂ 1.05 ^{ζ}CH₂ 1.53 ^{η}CH₂ 2.84 ^{τ1}CH₃ 2.70 |
| **Res. 5** | 9.88 | 5.47 | 1.57 | ^{τ1}CH₂ nd ^{τ2}CH₂ nd ^{τ3}CH₂ nd ^{τ4}CH nd |
| **Res. 6** | 9.77 | 5.52 | 1.57 | ^{τ1}CH₃ 2.72 |
| **Res. 7** | 9.58 | 5.37 | 2.03 | ^{ε}CH₂ 1.08 ^{ζ}CH₂ 1.53 ^{η}CH₂ 2.84 ^{τ1}CH₃ 2.67 |
| **Res. 8** | 9.80 | 5.54 | 1.59 | ^{τ1}CH₂ nd ^{τ2}CH₂ nd ^{τ3}CH₂ nd ^{τ4}CH nd |
| **Res. 9** | 9.72 | 5.61 | 1.57 | ^{τ1}CH₃ 2.64 |
| **NH₂** | 8.48 - 7.64 | - | - | - |

### 5. Oligomer 4

**4** was synthesized according to solid phase general procedures described in Section *VI.1.* starting from 0.4 mmol of of the corresponding resin-linked fully protected nonamer. Crude (≈ 0.20-0.25 mmol) was purified by preparative RP-HPLC to yield the pure peptide as a pulverulent white powder. Yield 19% (95 mg). Gradient (% of eluent B): 0→20 (3 min), 20→25 (3 min), 25→45 (20 min). LC Rt = 2.31 min. LC-MS: (ESI+): m/z 1268.3 ([M+2H]²⁺) 5%, 845.8 ([M+3H]³⁺) 35%, 634.3 ([M+4H]⁴⁺) 55%, 507.7 ([M+5H]⁵⁺) 95%, 423.3 ([M+6H]⁶⁺) 100%, 363.1 ([M+7H]⁷⁺) 55%.

### Results of the RP-HPLC are shown in Fig. 4.

The following table 2 represents the ¹HNMR chemical shifts for **4** in H₂O/D₂O (9:1) pH 6.5 at 283 K. The nomenclature of **4** is described Fig 10. **4** has the following formula :

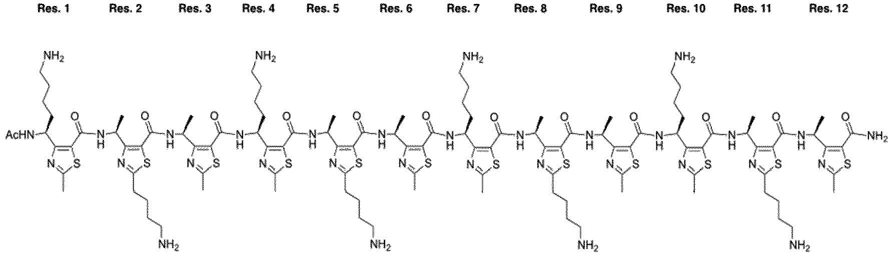

**[Table 2]**

| ***Residue*** | ***HN*** | ***^{γ}CH*** | ***^{δ}CH*** | ***Others*** |
|---|---|---|---|---|
| **Ac** | - | - | - | CH₃ 2.07 |
| **Res. 1** | 8.76 | 5.09 | 1.89 | ^{ε}CH₂ 0.92 ^{ζ}CH₂ 1.45 ^{η}CH₂ 2.78 |
| **Res. 2** | 10.13 | 5.49 | 1.56 | ^{τ1}CH₂ nd ^{τ2}CH₂ nd ^{τ3}CH₂ nd ^{τ4}CH nd |
| **Res. 3** | 9.91 | 5.54 | 1.57 | ^{τ1}CH₃ 2.73 |
| **Res. 4** | 9.65 | 5.38 | 2.05 | ^{ε}CH₂ 1.07 ^{ζ}CH₂ 1.56 ^{η}CH₂ 2.85 |
| **Res. 5** | 9.89 | 5.49 | 1.56 | ^{τ1}CH₂ nd ^{τ2}CH₂ nd ^{τ3}CH₂ nd ^{τ4}CH nd |
| **Res. 6** | 9.77 | 5.54 | 1.57 | ^{τ1}CH₃ 2.71 |
| **Res. 7** | 9.53 | 5.40 | 2.05 | ^{ε}CH₂ 1.07 □CH₂ 1.56 □CH₂ 2.85 |
| **Res. 8** | 9.86 | 5.51 | 1.56 | ^{τ1}CH₂ 3.03 - 3.05 ^{τ2}CH₂ 1.89 ^{τ3}CH₂ 1.80 ^{τ4}CH 2.97 |
| **Res. 9** | 9.62 | 5.53 | 1.56 | ^{τ1}CH₃ 2.65 |
| **Res. 10** | 9.53 | 5.40 | 2.05 | ^{ε}CH₂ 1.07 ^{ζ}CH₂ 1.56 ^{η}CH₂ 2.85 |
| **Res. 11** | 9.82 | 5.53 | 1.56 | ^{τ1}CH₂ nd ^{τ2}CH₂ nd ^{τ3}CH₂ nd ^{τ4}CH nd |
| **Res. 12** | 9.72 | 5.61 | 1.57 | ^{y}CH₃ 2.64 |
| **NH₂** | 8.45 - 7.62 | - | - | - |

### 7. Oligomer 6

**6** was synthesized according to solid phase general procedures described in Section *VI.1.* starting from 0.4 mmol of ChemMatrix Rink Amide resin. The crude was purified by preparative RP-HPLC to yield the pure peptide as a pulverulent white powder. Yield 6% (34 mg). Gradient (% of eluent B): 0→40 (2 min), 40→45 (3 min), 45→65 (20 min). LC Rt = 3.18 min. LC-MS: (ESI+): m/z 1084.6 ([M+2H]²⁺) 20%; 723.5 ([M+3H]³⁺) 70%, 542.9 ([M+4H]⁴⁺) 80%, 434.6 ([M+5H]⁵⁺) 100%, 362.2 ([M+6H]⁶⁺) 15%.

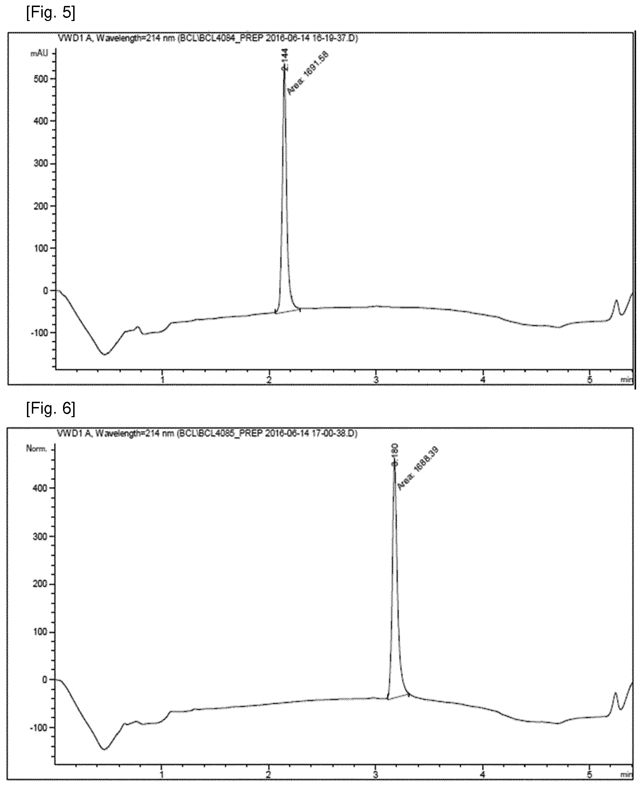

### Results of the RP-HPLC are shown in Fig. 6.

The following table 3 represents the ¹HNMR chemical shifts for **6** in CD3OH at 278 K. The nomenclature of **6** is described Fig 10. **6** has the following formula :

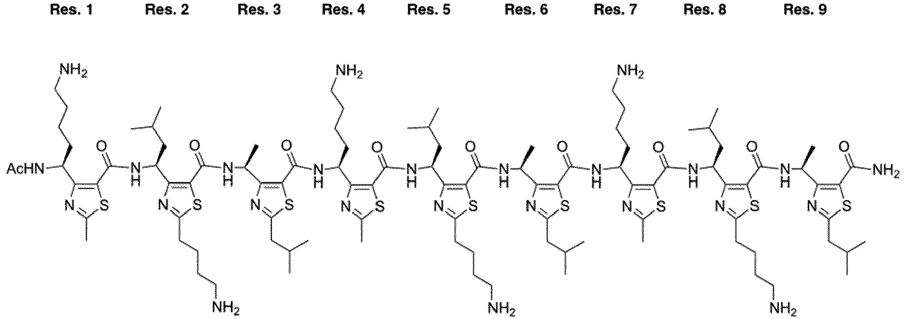

**[Table 3]**

| ***Residue*** | ***HN*** | ***^{γ}CH*** | ***^{δ}CH*** | ***Others*** |
|---|---|---|---|---|
| **Ac** | - | - | - | CH₃ 2.00 |
| **Res. 1** | 8.81 | 5.25 | 1.90 | ^{ε}CH₂ 1.02 - 1.13 ^{ζ}CH₂ 1.52 ^{η}CH₂ 2.77 ^{τ1}CH₃ 2.68 |
| **Res. 2** | 10.23 | 5.36 | 2.01 - 1.76 | ^{ε}CH₂ 1.05 ^{ζ1-2}CH₂ 0.56 - 0.81 ^{τ1}CH₂ 3.06 ^{τ2}CH₂ 1.89 ^{τ3}CH₂ 1.76 ^{τ4}CH 2.97 |
| **Res. 3** | 10.56 | 5.82 | 1.64 | ^{τ1}CH₃ 2.84 ^{τ2}CH₂ 2.10 ^{τ3}CH₂ 0.99 |
| **Res. 4** | 10.16 | 5.49 | 2.09 | ^{ε}CH₂ 1.21 - 1.33 ^{ζ}CH₂ 1.60 ^{η}CH₂ 2.84 ^{τ1}CH₃ 2.71 |
| **Res. 5** | 9.92 | 5.39 | 1.83 - 1.98 | ^{ε}CH₂ 1.18 ^{ζ1-2}CH₂ 0.63 - 0.82 ^{τ1}CH₂ 3.06 ^{τ2}CH₂ 1.89 ^{τ3}CH₂ 1.76 ^{τ4}CH 2.97 |
| **Res. 6** | 10.59 | 5.86 | 1.65 | ^{τ1}CH₃ 2.84 ^{τ2}CH₂ 2.10 ^{τ3}CH₂ 0.99 |
| **Res. 7** | 10.11 | 5.49 | 2.08 | ^{ε}CH₂ 1.23 - 1.33 ^{y}CH₂ 1.59 ^{y}CH₂ 2.82 ^{y}CH₃ 2.72 |
| **Res. 8** | 10.12 | 5.40 | 1.87-1.98 | ^{y}CH₂ 1.20 ^{y}CH₂ 0.63 - 0.83 ^{y}CH₂ 3.06 ^{y}CH₂ 1.89 ^{y}CH₂ 1.76 ^{y}CH 2.97 |
| **Res. 9** | 10.45 | 5.76 | 1.68 | ^{y}CH₃ 2.84 ^{y}CH₂ 2.10 ^{y}CH₂ 0.99 |
| **NH₂** | 8.74 - 7.70 | - | - | - |

### 8. Oligomer 7

**7** was synthesized according to solid phase general procedures described in Section *VI.1*. Crude was purified by preparative RP-HPLC to yield the pure peptide as a pulverulent white powder. Yield 5% (61 mg). Gradient (% of eluent B): 0→50 (5 min), 50→54 (2 min), 54→70 (16 min). LC Rt = 3.49 min. LC-MS: (ESI+): m/z 1436.5 ([M+2H]²⁺) 10%; 958.0 ([M+3H]³⁺) 40%, 718.8 ([M+4H]⁴⁺) 50%, 575.5 ([M+5H]⁵⁺) 95%, 479.5 ([M+6H]⁶⁺) 100%, 411.1 ([M+7H]⁷⁺) 40%, 359.7 ([M+8H]⁸⁺) 5%.

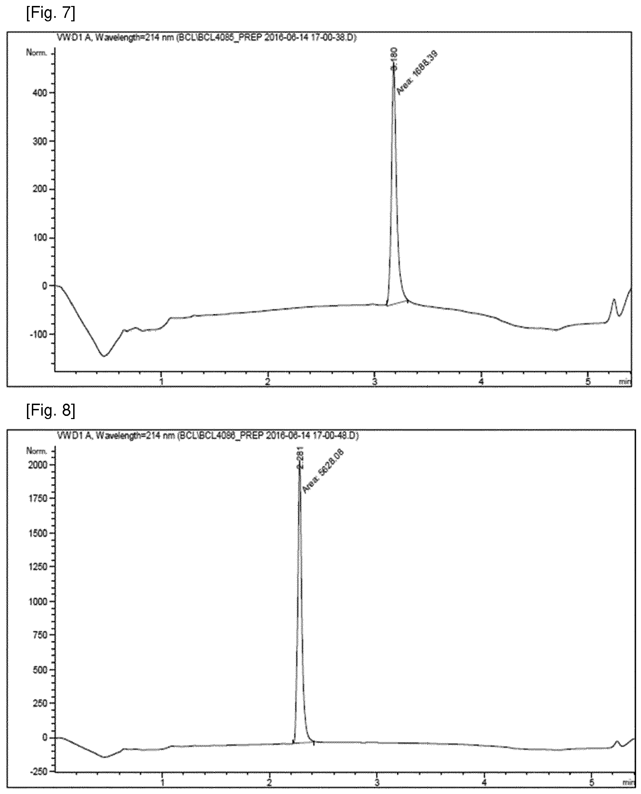

### Results of the RP-HPLC are shown in Fig. 7.

The following table 4 represents the ¹HNMR chemical shifts for 7 in CD₃OH at 278 K. The nomenclature of **7** is described Fig 10. **7** has the following formula :

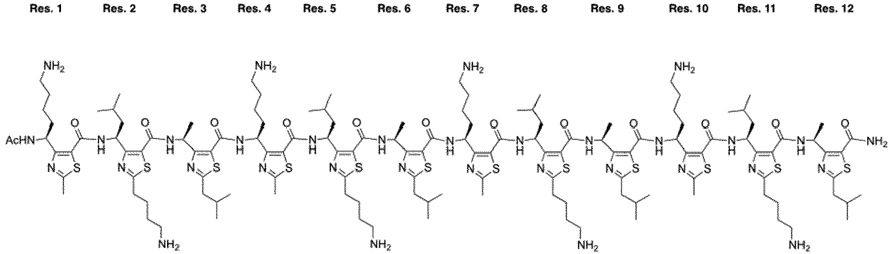

**[Table 4]**

| ***Residue*** | ***HN*** | ***^{γ}CH*** | ***^{δ}CH*** | ***Others*** |
|---|---|---|---|---|
| **Ac** | - | - | - | CH₃ 1.99 |
| **Res. 1** | 8.82 | 5.25 | 1.91 | ^{ε}CH₂ 1.02 - 1.13 ^{ζ}CH₂ 1.52 ^{η}CH₂ 2.76 ^{τ1}CH₃ nd |
| **Res. 2** | 10.25 | 5.35 | 2.03 - 1.77 | ^{y}CH₂ 1.06 ^{ζ1-2}CH₂ 0.56 - 0.81 ^{τ1}CH₂ 3.07 ^{τ2}CH₂ 1.89 ^{τ3}CH₂ 1.76 ^{τ4}CH 2.97 |
| **Res. 3** | 10.57 | 5.82 | 1.63 | ^{τ1}CH₃ 2.84 ^{τ2}CH₂ 2.10 ^{y}CH₂ 0.99 |
| **Res. 4** | 10.19 | 5.47 | 2.08 | ^{y}CH₂ 1.21 - 1.33 ^{y}CH₂ 1.61 ^{y}CH₂ 2.83 ^{y}CH₃ nd |
| **Res. 5** | 10.17 | 5.38 | 1.87 - 1.98 | ^{y}CH₂ 1.18 ^{y}CH₂ 0.64 - 0.84 ^{y}CH₂ 3.06 ^{y}CH₂ 1.89 ^{y}CH₂ 1.76 ^{y}CH 2.97 |
| **Res. 6** | 10.63 | 5.85 | 1.65 | ^{y}CH₃ 2.84 ^{y}CH₂ 2.10 ^{y}CH₂ 0.99 |
| **Res. 7** | 10.13 | 5.48 | 2.09 | ^{y}CH₂ 1.21 - 1.33 ^{y}CH₂ 1.62 ^{y}CH₂ 2.84 ^{y}CH₃ nd |
| **Res. 8** | 9.95 | 5.39 | 1.87 - 1.98 | ^{y}CH₂ 1.21 ^{y}CH₂ 0.64 - 0.84 ^{y}CH₂ 3.07 ^{y}CH₂ 1.89 ^{y}CH₂ 1.76 ^{y}CH 2.97 |
| **Res. 9** | 10.59 | 5.83 | 1.65 | ^{y}CH₃ 2.84 ^{y}CH₂ 2.10 ^{y}CH₂ 0.99 |
| **Res. 10** | 10.22 | 5.48 | 2.08 | ^{y}CH₂ 1.22 - 1.33 ^{y}CH₂ 1.60 ^{y}CH₂ 2.83 ^{y}CH₃ nd |
| **Res. 11** | 10.14 | 5.39 | 1.83 - 1.97 | ^{y}CH₂ 1.18 ^{y}CH₂ 0.64 - 0.83 ^{y}CH₂ 3.07 ^{y}CH₂ 1.89 ^{y}CH₂ 1.76 ^{y}CH 2.97 |
| **Res. 12** | 10.45 | 5.77 | 1.67 | ^{y}CH₃ 2.84 ^{y}CH₂ 2.10 ^{y}CH₂ 0.99 |
| **NH₂** | 8.75 - 7.70 | - | - | - |

### 9. Oligomer 8

**8** was synthesized according to solid phase general procedures described in Section *VI.1.* starting from 0.4 mmol of ChemMatrix Rink Amide resin. Crude was purified by preparative RP-HPLC to yield the pure peptide as a pulverulent white powder. Yield 5% (30 mg). Gradient (% of eluent B): 0→20 (3 min), 20→25 (2 min), 25→45 (25 min). LC Rt = 2.28 min. LC-MS: (ESI+): m/z 1043.0 ([M+2H]²⁺) 10%; 695.5 ([M+3H]³⁺) 40%, 521.8 ([M+4H]⁴⁺) 60%, 417.6 ([M+5H]⁵⁺) 100%, 348.2 ([M+6H]⁶⁺) 40%.

### Results of the RP-HPLC are shown in Fig. 8.

The following table 5 represents the ¹HNMR chemical shifts for **8** in CD₃OH at 278 K. The nomenclature of **8** is described Fig 10. **8** has the following formula :

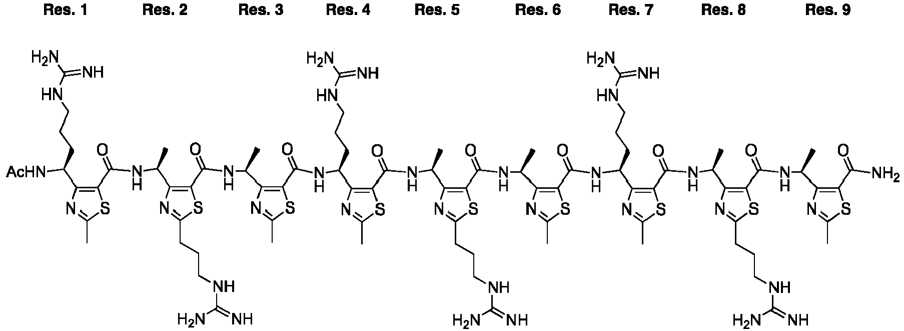

**[Table 5]**

| ***Residue*** | ***HN*** | ***^{γ}CH*** | ***^{δ}CH*** | ***Others*** |
|---|---|---|---|---|
| **Ac** | - | - | - | CH₃ 1.99 |
| **Res. 1** | 8.86 | 5.29 | 1.91 | ^{ε}CH₂ 1.36 - 1.19 ^{ζ}CH₂ 3.06 ^{τ1}CH₃ nd |
| **Res. 2** | 10.25 | 5.69 | 1.57 | ^{τ1}CH₂ 3.07 ^{y}CH₂ 2.07 ^{y}CH₂ 3.28 |
| **Res. 3** | 10.29 | 5.69 | 1.58 | ^{y}CH₃ nd |
| **Res. 4** | 10.22 | 5.53 | 2.08 | ^{y}CH₂ 1.54 - 1.38 ^{y}CH₂ 3.13 ^{y}CH₃ nd |
| **Res. 5** | 9.99 | 5.71 | 1.59 | ^{y}CH₂ 3.07 ^{y}CH₂ 2.07 ^{y}CH₂ 3.28 |
| **Res. 6** | 10.32 | 5.72 | 1.61 | ^{y}CH₃ 2.70 |
| **Res. 7** | 10.15 | 5.52 | 2.07 | ^{y}CH₂ 1.54 - 1.43 ^{y}CH₂ 3.14 ^{y}CH₃ nd |
| **Res. 8** | 9.77 | 5.75 | 1.60 | ^{y}CH₂ 3.07 ^{y}CH₂ 2.07 ^{y}CH₂ 3.28 |
| **Res. 9** | 10.19 | 5.65 | 1.64 | ^{y}CH₃ nd |
| **NH₂** | 8.62 - 7.67 | - | - | - |

### 10. Oligomer 9

**9** was synthesized according to solid phase general procedures described in Section *VI.1*.. Crude was purified by preparative RP-HPLC to yield the pure peptide as a pulverulent white powder. Yield 5% (37 mg). Gradient (% of eluent B): 0→25 (2 min), 25→28 (2 min), 28→48 (25 min). LC Rt = 2.35 min. LC-MS: (ESI+): m/z 920.5 ([M+3H]³⁺) 25%, 690.6 ([M+4H]⁴⁺) 70%, 552.5 ([M+5H]⁵⁺) 85%, 460.7 ([M+6H]⁶⁺) 100%, 395.0 ([M+7H]⁷⁺) 70%, 345.8 ([M+8H]⁸⁺) 15%.

### Results of the RP-HPLC are shown in Fig. 9.

The following table 6 represents the ¹HNMR chemical shifts for **9** in CD₃OH at 278 K. The nomenclature of **9** is described Fig 10. **9** has the following formula :

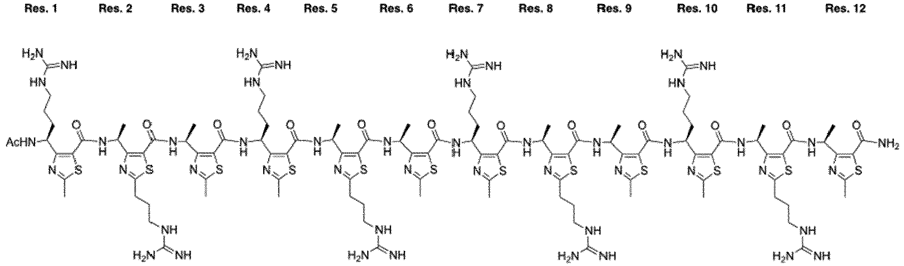

**[Table 6]**

| ***Residue*** | ***HN*** | ***^{γ}CH*** | ***^{δ}CH*** | ***Others*** |
|---|---|---|---|---|
| **Ac** | - | - | - | CH₃ 1.99 |
| **Res. 1** | 8.87 | 5.29 | 1.92 | ^{ε}CH₂ 1.36 - 1.19 ^{ζ}CH₂ 3.07 ^{τ1}CH₃ nd |
| **Res. 2** | 10.25 | 5.52 | 1.57 | ^{τ1}CH₂ 3.10 ^{τ2}CH₂ 2.07 ^{τ3}CH₂ 3.29 |
| **Res. 3** | 10.05 | 5.72 | 1.60 | ^{τ1}CH₃ nd |
| **Res. 4** | 10.26 | 5.69 | 2.08 | ^{ε}CH₂ 1.58 - 1.38 ^{ζ}CH₂ 3.14 ^{τ1}CH₃ nd |
| **Res. 5** | 10.30 | 5.68 | 1.59 | ^{τ1}CH₂ 3.10 ^{τ2}CH₂ 2.07 ^{τ3}CH₂ 3.29 |
| **Res. 6** | 10.37 | 5.71 | 1.62 | ^{τ1}CH₃ nd |
| **Res. 7** | 10.25 | 5.52 | 2.09 | ^{ε}CH₂ 1.56 - 1.39 ^{ζ}CH₂ 3.14 ^{τ1}CH₃ nd |
| **Res. 8** | 10.00 | 5.71 | 1.61 | ^{τ1}CH₂ 3.10 ^{τ2}CH₂ 2.07 ^{τ3}CH₂ 3.29 |
| **Res. 9** | 10.33 | 5.73 | 1.61 | ^{τ1}CH₃ nd |
| **Res. 10** | 10.15 | 5.53 | 2.07 | ^{ε}CH₂ 1.55 - 1.43 ^{ζ}CH₂ 3.14 ^{τ1}CH₃ nd |
| **Res. 11** | 9.78 | 5.75 | 1.60 | ^{τ1}CH₂ 3.10 ^{τ2}CH₂ 2.07 ^{τ3}CH₂ 3.29 |
| **Res. 12** | 10.19 | 5.65 | 1.64 | ^{τ1}CH₃ nd |
| **NH₂** | 8.62 - 7.67 | - | - | - |

### IV. Synthesis of scrambled sequence 5 (helical but not amphipathic/amphiphilic)

**5** was synthesized according to solid phase general procedures described in Section *VI.1.* starting from 0.15 mmol of ChemMatrix Rink Amide resin. Crude was purified by preparative RP-HPLC to yield **5** as a pulverulent white powder. Yield 18% (92 mg). Gradient (% of eluent B): 0→15 (2 min), 15→20 (3 min), 20→40 (25 min). LC Rt = 2.14 min. LC-MS: (ESI+): m/z 845.4 ([M+3H]³⁺) 30%, 634.4 ([M+4H]⁴⁺) 55%, 507.9 ([M+5H]⁵⁺) 80%, 423.3 ([M+6H]⁶⁺) 100%, 362.9 ([M+7H]⁷⁺) 50%.

Results of the RP-HPLC are shown in Fig. 5.

### EXAMPLE 2 - Structural analysis of the polycationic and facially amphiphilic helical molecular pseudo-peptide architectures.

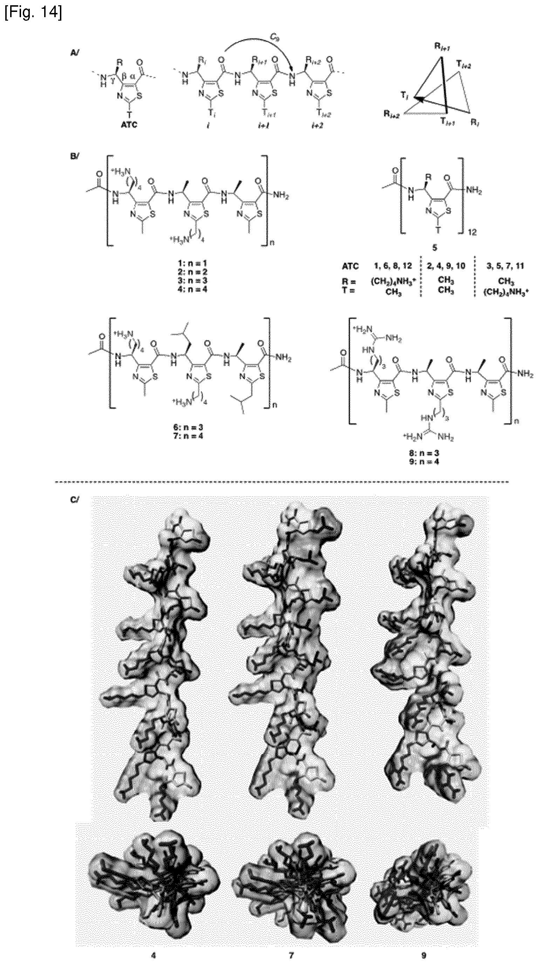

Previous structural investigations have demonstrated that ATC oligomers appeared to be closed to a C₃-symmetry helix with three residues to achieve a complete rotation. The architecture is stabilized by a highly stable hydrogen-bonding pattern between residues *i* and *i+2* and exhibits six substituents per turn distributed around a 60° angle along the axis. Side chain positioning defines three distinct faces over the γ-peptide (**Fig. 14** **A**/). Substituents R₁, R₁' as defined above share the first face. Substituents R₂, R₂' as defined above share the second face. Substituents R₃, R₃' as defined above share the third face. On the basis of this topology, sequences **1-4** and **6-9 (****Fig 14B****/)** were designed to display one polycationic face and two hydrophobic faces (**Fig. 14** **C**/). Oligomer 5 was designed as a scrambled sequence that does not display segregation of the cationic charges along one face of the helical architecture.

Circular dichroism (CD), NMR and FT-IR structural markers of the helical conformation of thiazole-based γ-peptides named ATC-peptides have been previously reported (C. Bonnel, B. Legrand, J. L. Bantignies, H. Petitjean, J. Martinez, N. Masurier, L. T. Maillard, Org. Biomol. Chem. 2016, 14, 8664-8669*;* L. Mathieu, B. Legrand, C. Deng, L. Vezenkov, E. Wenger, C. Didierjean, M. Amblard, M. C. Averlant-Petit, N. Masurier, V. Lisowski, J. Martinez, L. T. Maillard, Angew. Chem., Int. Ed. Engl. 2013, 52, 6006-6010*).*

**Procedures for NMR experiments: 3, 4** (2-4 mM) were dissolved in H₂0/D₂O(pH 6.5) while **6, 7, 8** and **9** (2-4 mM) were dissolved in CD₃OH. All spectra were recorded on a Bruker Avance 600 AVANCE III spectrometer equipped with a 5 mm triple-resonance cryoprobe (1H, 13C, 15N). Homonuclear 2-D spectra DQF-COSY, TOCSY (DIPSI2) and ROESY were typically recorded in the phase-sensitive mode using the States-TPPI method as data matrices of 256-512 real (t1) x 2048 (t2) complex data points; 8-48 scans per t1 increment with 1.0 s recovery delay and spectral width of 6009 Hz in both dimensions were used. The mixing times were 60 ms for TOCSY and 350-450 ms for the ROESY experiments. In addition, 2D heteronuclear spectra 15N, 13C-HSQC and 13C-HSQC-TOCSY were acquired to fully assign the oligomers (8-32 scans, 256-512 real (t1) × 2048 (t2) complex data points). Spectra were processed with Topspin (Bruker Biospin) and visualized with Topspin or NMRview on a Linux station. The matrices were zero-filled to 1024 (t1) x 2048 (t2) points after apodization by shifted sine-square multiplication and linear prediction in the F1 domain. Chemical shifts were referenced to the solvent.

**Procedures for molecular modeling studies:** ¹H chemical shifts were assigned according to classical procedures. NOE cross-peaks were integrated and assigned within the NMRView software (B. A. Johnson, R. A. Blevins, J. Biomol. NMR 1994, 4, 603 1994, 4). The volume of a ROE between methylene pair protons was used as a reference of 1.8 Å. The lower bound for all restraints was fixed at 1.8 Å and upper bounds at 2.7, 3.3 and 5.0 Å, for strong, medium and weak correlations, respectively (K. Wüthrich, NMR of Proteins and Nucleic acids; Wiley-Interscience: New York, 1986*)* Pseudo-atoms corrections of the upper bounds were applied for unresolved aromatic, methylene and methyl protons signals. Structure calculations were performed with AMBER 16 (D. A. Case, T. A. Darden, T. E. Cheatham III, C. L. Simmerling, J. Wang, R. E. Duke, R. Luo, M. Crowley, R. C. Walker, W. Zhang, K. M. Merz, B. Wang, S. Hayik, A. Roitberg, G. Seabra, I. Kolossvry, K. F. Wong, F. Paesani, J. Vanicek, X. Wu, S. R. Brozell, T. Steinbrecher, H. Gohlke, L. Yang, C. Tan, J. Mongan, V. Hornak, G. Cui, D. H. Mathews, M. G. Seetin, C. Sagui, V. Babin and P. A. Kollman, Amber 10, University of California, San Francisco, 2008) in two stages: cooking and simulated annealing with the generalized Born (GB) implicit solvent model. The cooking stage was performed at 1000 K to generate 100 initial random structures. SA calculations were carried during 20 ps (20000 steps, 1 fs long) as described elsewhere. First, the temperature was risen quickly and was maintained at 600 K for the first 5000 steps, then the system was cooled gradually from 600 K to 100 K from step 5001 to 18000 and finally the temperature was brought to 0 K during the 2000 remaining steps. For the 3000 first steps, the force constant of the distance restraints was increased gradually from 2.0 kcal.mol-1.Å to 20 kcal.mol-1.Å. For the rest of the simulation (step 3001 to 20000), the force constant is kept at 20 kcal.mol-1.Å. The calculations were launched in vacuum. The 10 lowest energy structures with no violations > 0.3 Å were considered as representative of the peptide structure. The representation and quantitative analysis were carried out using MOLMOL(R. Koradi, M. Billeter, K. Wuthrich, J. Mol. Graph. 1996, 14, 51) and Chimera *(*Delano Scientific Pettersen EF, Goddard TD, Huang CC, Couch GS, Greenblatt DM, Meng EC, Ferrin TE. J. Comput. Chem. 2004, 25, 1605-1612*).*

### Procedure for Circular dichroism (CD) experiments:

CD were carried out using a Jasco J815 spectropolarimeter. The spectra were obtained in water, pH 6.0 using a 1 mm path length CD cuvette, at 20°C, over a wavelength range of 190-300 nm. Continuous scanning mode was used, with a response of 1.0 s with 0.2 nm steps and a bandwidth of 2 nm. The signal to noise ratio was improved by acquiring each spectrum over an average of two scans. Baseline was corrected by subtracting the background from the sample spectrum. The samples were dissolved in water at 25 µM.

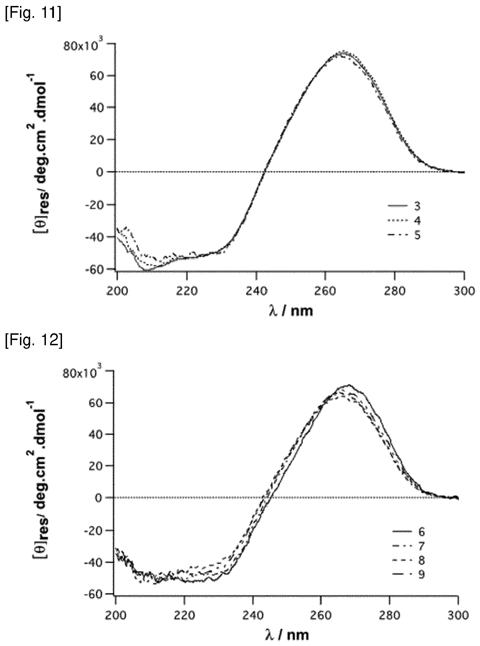

Fig. 11 and 12 represents CD spectra of oligomers (3), (4), (5), and (6), (7), (8) and (9) in water at 20°C, respectively.

### Procedure for FT-IR experiments:.

Middle-infrared experiments (1000-2000 cm⁻¹) were recorded in the ATR mode equipped with a diamond crystal. The measurements were carried out on a Perkin-Elmer Spectrum one FT-IR spectrometer equipped with a deuterated (L)-alanine triglycine sulphate (FR-DTGS) pyroelectric detector, a Globar source, and potassium bromide (KBr) beam splitter. The spectral resolution was 4 cm-1, and 64 scans were co-added for each spectrum. The compounds were dissolved at 5 mM concentration in water and 10µl solutions were deposited on the ATR crystal. The FT-IR spectra were smoothed. Baseline substraction and deconvolution were performed using IGOR Pro 6.0 software (WaveMetrics). The deconvolution using a sum of Gaussian functions (in dash lines) and the overall fit (in red) are also shown.

### Evidences of the facially amphiphilic morphology of the oligomer 3:

The far-UV CD spectrum of **3** was recorded in water (pH 6.0) at 25 µM between 200 and 300 nm. With two minima at 208 and 220 nm and a strong maximum at 265 nm, the CD signature is consistent with a Cg-helical folding.(C. Bonnel, B. Legrand, J. L. Bantignies, H. Petitjean, J. Martinez, N. Masurier, L. T. Maillard, Org. Biomol. Chem. 2016, 14, 8664-8669*)* In addition, the molar ellipticity value per residue (73600 deg.cm⁻² dmol⁻¹ for 3 at 265 nm) was similar to the previously value (74000 deg.cm⁻².dmol⁻¹) reported for helical ATC-oligomers in water.

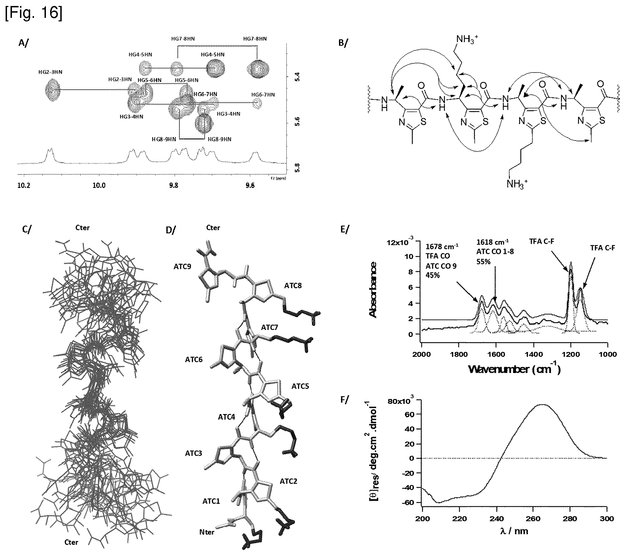

NMR analyses of **3** were conducted in H₂O/D₂O (9:1), pH 6.5. As previously observed on ATCs-containing oligomers, (L. Mathieu, B. Legrand, C. Deng, L. Vezenkov, E. Wenger, C. Didierjean, M. Amblard, M. C. Averlant-Petit, N. Masurier, V. Lisowski, J. Martinez, L. T. Maillard, Angew. Chem., Int. Ed. Engl. 2013, 52, 6006-6010*;* B. Legrand, L. Mathieu, A. Lebrun, S. Andriamanarivo, V. Lisowski, N. Masurier, S. Zirah, Y. K. Kang, J. Martinez, L. T. Maillard, Chem. Eur. J. 2014, 20, 6713-6720*)* all the amide protons signals were strongly downfield, i.e. from 8.87 to 10.13 ppm. Such a strong NH deshielding (> 9 ppm) has been recognized as a structural marker related to the formation of a C9 hydrogen-bonding pattern. (C. Bonnel, B. Legrand, J. L. Bantignies, H. Petitjean, J. Martinez, N. Masurier, L. T. Maillard, Org. Biomol. Chem. 2016, 14, 8664-8669*)* In addition, 3J(NH,γCH) values < 6 Hz (5.3 ± 0.3 Hz) were typical of φ values around -60° as expected for a Cg-helix. Further evidences of Cg-helical folding came from the analysis of the 2D-ROESY spectra, which revealed characteristic medium sequential NH(i)/γCH(i-1) and weak γCH(i-1)/γCH(i) correlations. Many weak NH(i)/ γCH(i-1), NH(i)/γCH(i-1) and γCH(i-1)/δCH(i) NOE connectivities recognized as structural markers of the ATC helix were also visible for **3** (**Fig. 16****/A-B**). (C. Bonnel, B. Legrand, J. L. Bantignies, H. Petitjean, J. Martinez, N. Masurier, L. T. Maillard, Org. Biomol. Chem. 2016, 14, 8664-8669) NOEs were used as restraints for NMR solution structure calculations of 3 using a simulated annealing protocol with AMBER 16. Unambiguous 99 distance restraints were introduced to generate an ensemble of 15-lowest energy structures in water that converged toward a well-defined Cg-helix in which all the cationic lateral chains were distributed along a single face (**Fig 16****/C**). (C. Bonnel, B. Legrand, J. L. Bantignies, H. Petitjean, J. Martinez, N. Masurier, L. T. Maillard, Org. Biomol. Chem. 2016, 14, 8664-8669)

FTIR experiments also confirmed the amide hydrogen bonding state of 3 in water. The amide I frequencies provide unambiguous structural indicators of the H-bond network for ATC-containing oligomers. (C. Bonnel, B. Legrand, J. L. Bantignies, H. Petitjean, J. Martinez, N. Masurier, L. T. Maillard, Org. Biomol. Chem. 2016, 14, 8664-8669*)* The band at 1618 cm⁻¹ is attributed to the bound ATC C=O. No band is observed around 1645 cm⁻¹, which confirms that most of the ATC carbonyls is involved in hydrogen-bonds.

Taking all together, the NMR, FT-IR and CD data were strong evidences of the facially amphiphilic morphology of the designed γ-peptides.

### Evidences of the facially amphiphilic morphology of the oligomer 4:

The far-UV CD spectrum of **4** was recorded in water (pH 6.0) at 25 µM between 200 and 300 nm. With two minima at 208 and 220 nm and a strong maximum at 265 nm, the CD signature is consistent with a Cg-helical folding.(C. Bonnel, B. Legrand, J. L. Bantignies, H. Petitjean, J. Martinez, N. Masurier, L. T. Maillard, Org. Biomol. Chem. 2016, 14, 8664-8669*)* In addition, the molar ellipticity value per residue (73600 deg.cm⁻² dmol⁻¹ for 3 at 265 nm) was similar to the previously value (74000 deg.cm⁻².dmol⁻¹) reported for helical ATC-oligomers in water.

NMR analyses of **4** were conducted in H₂O/D₂O (9:1), pH 6.5. As previously observed on ATCs-containing oligomers, (C. Bonnel, B. Legrand, J. L. Bantignies, H. Petitjean, J. Martinez, N. Masurier, L. T. Maillard, Org. Biomol. Chem. 2016, 14, 8664-8669*;* B. Legrand, L. Mathieu, A. Lebrun, S. Andriamanarivo, V. Lisowski, N. Masurier, S. Zirah, Y. K. Kang, J. Martinez, L. T. Maillard, Chem. Eur. J. 2014, 20, 6713-6720*)* all the amide protons signals were strongly downfield, i.e. from 8.76 to 10.13 ppm. Such a strong NH deshielding (> 9 ppm) has been recognized as a structural marker related to the formation of a C9 hydrogen-bonding pattern. In addition, 3J(NH,γCH) values < 6 Hz (5.3 ± 0.3 Hz) were typical of φ values around -60° as expected for a Cg-helix. Further evidences of Cg-helical folding came from the analysis of the 2D-ROESY spectra, which revealed characteristic medium sequential NH(i)/γCH(i-1) and weak γCH(i-1)/ γCH(i) correlations.

FTIR experiments also confirmed the amide hydrogen bonding state of **4** in water. The amide I frequencies provide unambiguous structural indicators of the H-bond network for ATC-containing oligomers.(C. Bonnel, B. Legrand, J. L. Bantignies, H. Petitjean, J. Martinez, N. Masurier, L. T. Maillard, Org. Biomol. Chem. 2016, 14, 8664-8669*)* The band at 1618 cm⁻¹ is attributed to the bound ATC C=O. No band is observed around 1645 cm⁻¹, which confirms that most of the ATC carbonyls is involved in hydrogen-bonds.

Taking all together, the NMR, FT-IR and CD data were strong evidences of the facially amphiphilic morphology of **4.**

### Evidences of the helical conformation of the oligomer 5:

The helical shape of **5** was confirmed by CD and FT-IR experiments. 1/ the CD signatures and the molar ellipticity values per residue were identical to those of **3** and **4**. 2/ The FT-IR spectrum in the 1000 - 2000 cm⁻¹ region was readily similar to those of **3** and 4 without any band around 1645 cm⁻¹ as expected for folded oligomers.

Table 7 represents Coupling Constants ³J(NH, ^{γ}CH) of **3, 4, 6, 7, 8** and **9** (in Hz). Values were measured in H₂0/D₂O(pH 6.5) for **3, 4** and **5** and in CD₃OH for **6, 7, 8** and **9.**

**[Table 7]**

| **Residue** | **3** | **4** | **6** | **7** | **8** | **9** |
|---|---|---|---|---|---|---|
| | (283 K) | (283 K) | (278 K) | (278 K) | (278 K) | (278 K) |
| **Res. 1** | **5.6** | **5.5** | 7.0 | 7.0 | 7.0 | 6.9 |
| **Res. 2** | **5.4** | **5.4** | 5.0 | o | 5.1 | 5.0 |
| **Res. 3** | **5.2** | **5.3** | 5.8 | 6.1 | 5.1 | 5.9 |
| **Res. 4** | **5.3** | **5.1** | 5.8 | o | 6.6 | 5.0 |
| **Res. 5** | **5.0** | **5.7** | 6.0 | o | 5.8 | 5.0 |
| **Res. 6** | **5.0** | **4.9** | 6.0 | o | 5.1 | 5.0 |
| **Res. 7** | **5.1** | **5.2** | 6.0 | o | 6.6 | 5.0 |
| **Res. 8** | **5.2** | **4.7** | 6.0 | 6.1 | 6.2 | 5.6 |
| **Res. 9** | **5.9** | **4.9** | 6.9 | 5.1 | 6.6 | 5.6 |
| **Res. 10** | | **5.2** | | o | | 5.6 |
| **Res. 11** | | **5.1** | | o | | 6.3 |
| **Res. 12** | | **6.2** | | 6.6 | | 6.3 |

Table 8 represents Inter-residue NOE correlations observed in the ROESY spectrum of (**3**) and (**4**) in H₂O/D₂O (9:1) pH 6.5 at 283 K.

**[Table 8]**

| **Compound 3** | | **Compound 4** | | |
|---|---|---|---|---|
| **NOE correlations** | **Intensity** | **NOE correlations** | **Intensity** | **Type** |
| Ac-2.NH | w | Ac-2.NH | w | Long range |
| 1.NH-2.NH | w | 1.NH-2.NH | nd | Sequential |
| 1.Hγ-2.NH | m | 1.Hγ-2.NH | m | Sequential |
| 1.Hγ-2.Hγ | w | 1.Hγ-2.Hγ | nd | Sequential |
| 1.Hγ-2.Hδ | w | 1.Hγ-2.Hδ | overlap | Sequential |
| 1.Hδ-2.NH | w | 1.Hδ-2.NH | w | Sequential |
| 1.Hε-2.NH | w | 1.Hε-2.NH | nd | Sequential |
| 2.NH-3.NH | w | 2.NH-3.NH | nd | Sequential |
| 2.Hγ-3.NH | m | 2.Hγ-3.NH | m | Sequential |
| 2.Hγ-3.Hγ | w | 2.Hγ-3.Hγ | nd | Sequential |
| 2.Hγ-3.Hδ | w | 2.Hγ-3.Hδ | overlap | Sequential |
| 2.Hδ-3.NH | w | 2.Hδ-3.NH | overlap | Sequential |
| 2.Hγ-3.CH₃(thiazole) | w | 2.Hγ-3.CH₃(thiazole) | nd | Sequential |
| 3.NH-4.NH | w | 3.NH-4.NH | nd | Sequential |
| 3.Hγ-4.NH | m | 3.Hγ-4.NH | m | Sequential |
| 3.Hγ-4.Hγ | w | 3.Hγ-4.Hγ | overlap | Sequential |
| 3.Hγ-4.Hδ | w | 3.Hγ-4.Hδ | overlap | Sequential |
| 3.Hδ-4.NH | w | 3.Hδ-4.NH | overlap | Sequential |
| 3.Hγ-4.CH₃(thiazole) | w | 3.Hγ-4.CH₃(thiazole) | nd | Sequential |
| 4.NH-5.NH | w | 4.NH-5.NH | nd | Sequential |
| 4.Hγ-5.NH | m | 4.Hγ-5.NH | m | Sequential |
| 4.Hδ-5.NH | w | 4.Hδ-5.NH | w | Sequential |
| 4.Hε-5.NH | w | 4.Hε-5.NH | w | Sequential |
| 4.Hγ-5.Hγ | w | 4.Hγ-5.Hγ | nd | Sequential |
| 4.Hγ-5.Hδ | w | 4.Hγ-5.Hδ | overlap | Sequential |
| 5.NH-6.NH | w | 5.NH-6.NH | nd | Sequential |
| 5.Hγ-6.NH | m | 5.Hγ-6.NH | m | Sequential |
| 5.Hγ-6.Hγ | w | 5.Hγ-6.Hγ | nd | Sequential |
| 5.Hγ-6.Hδ | w | 5.Hγ-6.Hδ | overlap | Sequential |
| 5.Hδ-6.NH | w | 5.Hδ-6.NH | overlap | Sequential |
| 5.Hγ-6.CH₃(thiazole) | w | 5.Hγ-6.CH₃(thiazole) | nd | Sequential |
| 6.NH-7.NH | w | 6.NH-7.NH | nd | Sequential |
| 6.Hγ-7.NH | m | 6.Hγ-7.NH | m | Sequential |
| 6.Hγ-7.Hγ | w | 6.Hγ-7.Hγ | overlap | Sequential |
| 6.Hγ-7.Hδ | w | 6.Hγ-7.Hδ | overlap | Sequential |
| 6.Hδ-7.NH | w | 6.Hδ-7.NH | overlap | Sequential |
| 6.Hγ-7.CH₃(thiazole) | w | 6.Hγ-7.CH₃(thiazole) | nd | Sequential |
| 7.NH-8.NH | w | 7.NH-8.NH | nd | Sequential |
| 7.Hγ-8.NH | m | 7.Hγ-8.NH | m | Sequential |
| 7.Hγ-8.Hγ | w | 7.Hγ-8.Hγ | nd | Sequential |
| 7.Hγ-8.Hδ | w | 7.Hγ-8.Hδ | overlap | Sequential |
| 7.Hδ-8.NH | w | 7.Hδ-8.NH | w | Sequential |
| 7.Hε-8.NH | w | 7.Hε-8.NH | nd | Sequential |
| 8.NH-9.NH | nd | 8.NH-9.NH | nd | Sequential |
| 8.Hγ-9.NH | m | 8.Hγ-9.NH | m | Sequential |
| 8.Hγ-9.Hγ | nd | 8.Hγ-9.Hγ | nd | Sequential |
| 8.Hγ-9.Hδ | w | 8.Hγ-9.Hδ | overlap | Sequential |
| 8.Hδ-9.NH | w | 8.Hδ-9.NH | overlap | Sequential |
| 8.Hγ-9.CH₃(thiazole) | w | 8.Hγ-9.CH₃(thiazole) | nd | Sequential |
| 9.NH-NH₂ | w | 9.NH-10.NH | nd | Sequential |
| 9.Hγ-NH₂ | w | 9.Hγ-10.NH | m | Sequential |
| 9.Hδ-NH₂ | w | 9.Hδ-10.NH | overlap | Sequential |
| | | 9.Hγ-10.Hγ | overlap | Sequential |
| | | 10.NH-11.NH | nd | Sequential |
| | | 10.Hγ-11.NH | m | Sequential |
| | | 10.Hδ-11.NH | w | Sequential |
| | | 10.Hγ-11.Hγ | nd | Sequential |
| | | 11.NH-12.NH | nd | Sequential |
| | | 11.Hγ-12.NH | m | Sequential |
| | | 11.Hγ-12.Hγ | nd | Sequential |
| | | 12.NH-NH₂ | nd | Sequential |
| | | 12.Hγ-NH₂ | w | Sequential |

Observed NOE connectivities that could not be attributed unambiguously are shown below for compound 4 :

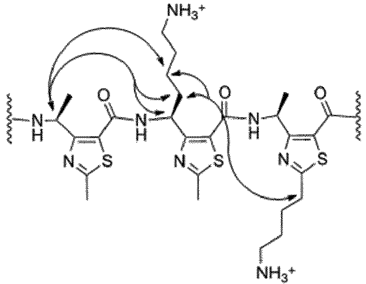

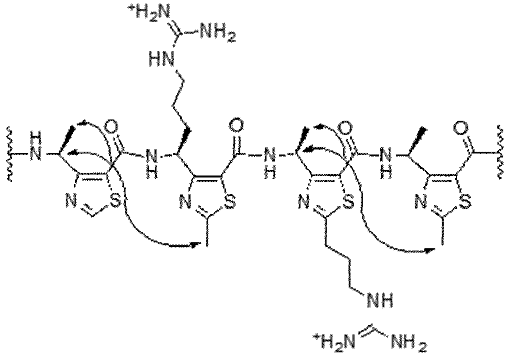

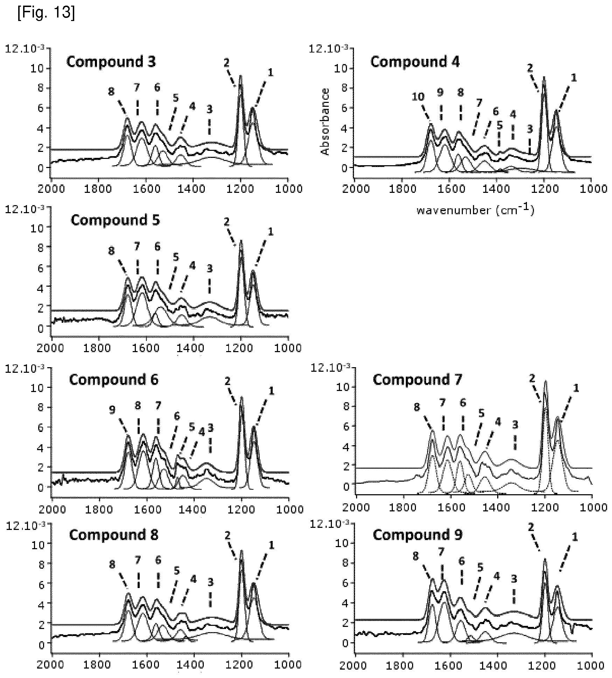

Figures 13A-13G represent FT-IR spectra (1000-2000 cm-1) of oligomers (3), (4), (5), (6), (7), (8) and (9) in water at 20°C, respectively. Table 11 represents band positions, band intensities, band widths and band areas in the region of 1000-2000 cm⁻¹.

**[Table 11]**

| **Compounds** | **Absorption bands** | **Wave-numbers** | **Intensity (a.u.)** | **Width (a.u.)** | **Area (a.u.)** |
|---|---|---|---|---|---|
| **Compound 3 TFA salt** | 1 (TFA C-F) | 1147 | 0.0041 | 27.9 | 0.207 |
| | 2 (TFA C-F) | 1198 | 0.0072 | 16.0 | 0.206 |
| | 3 | 1321 | 0.0007 | 76.8 | 0.098 |
| | 4 | 1452 | 0.0012 | 28.0 | 0.058 |
| | 5 | 1529 | 0.0014 | 26.8 | 0.068 |
| | 6 | 1560 | 0.0020 | 20.2 | 0.073 |
| | 7 | 1617 | 0.0027 | 31.0 | 0.151 |
| | 8 | 1678 | 0.0030 | 22.7 | 0.124 |
| | | | | | |
| **Compound 4 TFA salt** | 1 (TFA C-F) | 1147 | 0.0046 | 25.6 | 0.208 |
| | 2 (TFA C-F) | 1198 | 0.0079 | 16.0 | 0.225 |
| | 3 | 1293 | 0.0003 | 73.7 | 0.048 |
| | 4 | 1340 | 0.0006 | 33.3 | 0.035 |
| | 5 | 1384 | 0.0003 | 9.4 | 0.005 |
| | 6 | 1451 | 0.0011 | 33.7 | 0.067 |
| | 7 | 1532 | 0.0015 | 29.9 | 0.081 |
| | 8 | 1562 | 0.0018 | 19.3 | 0.062 |
| | 9 | 1618 | 0.0028 | 32.2 | 0.158 |
| | 10 | 1678 | 0.0032 | 23.1 | 0.132 |
| | | | | | |
| **Compound 5 TFA salt** | 1 (TFA C-F) | 1147 | 0.0040 | 21.6 | 0.209 |
| | 2 (TFA C-F) | 1198 | 0.0070 | 16.1 | 0.201 |
| | 3 | 11332 | 0.0008 | 54.5 | 0.080 |
| | 4 | 1451 | 0.0012 | 30.8 | 0.066 |
| | 5 | 1540 | 0.0018 | 40.3 | 0.132 |
| | 6 | 11562 | 0.0013 | 16.6 | 0.039 |
| | 7 | 1619 | 0.0033 | 32.1 | 0.190 |
| | 8 | 11678 | 0.0031 | 21.4 | 0.121 |
| | | | | | |
| **Compound 6 TFA salt** | 1 (TFA C-F) | 1147 | 0.0046 | 21.5 | 0.177 |
| | 2 (TFA C-F) | 1198 | 0.0076 | 15.8 | 0.213 |
| | 3 | 1347 | 0.0009 | 40.0 | 0.066 |
| | 4 | 1448 | 0.0014 | 26.0 | 0.067 |
| | 5 | 1470 | 0.0011 | 9.2 | 0.018 |
| | 6 | 1529 | 0.0020 | 26.4 | 0.094 |
| | 7 | 1562 | 0.0030 | 18.8 | 0.102 |
| | 8 | 1616 | 0.0039 | 19.2 | 0.200 |
| | 9 | 1678 | 0.0037 | 21.4 | 0.140 |
| **Compound 7 TFA salt** | 1 (TFA C-F) | 1147 | 0.0053 | 28.5 | 0.269 |
| | 2 (TFA C-F) | 1198 | 0.0088 | 16.4 | 0.258 |
| | 3 | 1343 | 0.0009 | 46.8 | 0.075 |
| | 4 | 1456 | 0.0017 | 29.6 | 0.091 |
| | 5 | 1526 | 0.0018 | 21.6 | 0.070 |
| | 6 | 1561 | 0.0033 | 20.5 | 0.120 |
| | 7 | 1616 | 0.0034 | 27.1 | 0.161 |
| | 8 | 1678 | 0.0039 | 22.5 | 0.156 |
| | | | | | |
| **Compound 8 TFA salt** | 1 (TFA C-F) | 1147 | 0.0041 | 28.1 | 0.207 |
| | 2 (TFA C-F) | 1198 | 0.0072 | 16.0 | 0.205 |
| | 3 | 1322 | 0.0007 | 80.5 | 0.102 |
| | 4 | 1435 | 0.0005 | 12.0 | 0.010 |
| | 5 | 1458 | 0.0010 | 24.2 | 0.045 |
| | 6 | 1533 | 0.0016 | 30.1 | 0.083 |
| | 7 | 1562 | 0.0017 | 18.8 | 0.057 |
| | 8 | 1617 | 0.0027 | 31.4 | 0.153 |
| | 9 | 1678 | 0.0031 | 22.7 | 0.124 |
| | | | | | |
| **Compound 9 TFA salt** | 1 (TFA C-F) | 1147 | 0.0034 | 43.7 | 0.158 |
| | 2 (TFA C-F) | 1198 | 0.0060 | 26.2 | 0.169 |
| | 3 | 1330 | 0.0008 | 125.4 | 0.108 |
| | 4 | 1452 | 0.0011 | 48.3 | 0.058 |
| | 5 | 1514 | 0.0006 | 27.4 | 0.017 |
| | 6 | 1556 | 0.0022 | 44.1 | 0.104 |
| | 7 | 1625 | 0.0040 | 51.5 | 0.219 |
| | 8 | 1677 | 0.0038 | 34.5 | 0.140 |

### EXAMPLE 3 - Biological properties

### Minimal inhibitory concentration (MIC) values and minimal bactericidal concentration (MBCs)

Minimal inhibitory concentration (MIC) values and minimal bactericidal concentration (MBCs) values for ATC-peptides **1-9** were determined against both Gram-positive (*Bacillus subtilis, Staphylococcus aureus, Enterococcus faecalis*) and Gram negative (*Escherichia coli, Pseudomonas aeruginosa*) bacteria as well as a yeast (*Candida albicans*)*.* Experiments were conducted in triplicate. For B. *subtilis,* compounds were tested against vegetative cells and spores, which are particularly resistant to the action of antimicrobial agents. MIC and MBCs values are shown **Table 12.** In addition, haemolytic properties of 1-9 were tested.

The scrambled sequence **5** was less active than its amphiphilic counterpart **4.**

**[Table 12]**

| Sequen ce | *B*. *subtilis* ATCC66 33^{[a]} | *S*. *aureus* ATCC6538^{[ a]} | *E. faecalis* ATCC29121 [a] | *E. coli* ATCC8739^{[ a]} | P. *aeruginosa* ATCC9027^{[ a]} | *C. albicans* ATCC10231 ^{[a]} | HD₅₀^{[ b]} |
|---|---|---|---|---|---|---|---|
| **1** | >150 (>150) | >150 (>150) | >150 (>150) | >150 (>150) | >150 (>150) | >150 (>150) | > 100 |
| **2** | 30 (30) | >150 (>150) | >150 (>150) | >150 (>150) | >150 (>150) | 62.5 (130) | > 100 |
| **3** | 0.8 (0.8) | >150 (>150) | >150 (>150) | 5.2 (7.3) | 42 (83) | 10.4 (10.4) | > 100 |
| **4** | 0.5 (0.5) | 26 (57) | >150 (>150) | 1.6 (1.6) | 3.9 (5.2) | 3.7 (3.7) | > 100 |
| **5** | 0.8 (nd) | 100 (nd) | >150 (nd) | 5.2 (nd) | 100 (nd) | 50 (nd) | HD₁₀ 62.5 |
| **6** | 1.3 (1.8) | 5.2 (5.2) | 6.2 (6.2) | 8.3 (8.3) | 6.2 (6.2) | 6.2 (6.2) | 23 |
| **7** | 4.2 (6.2) | 16.7 (25) | 10.4 (12.5) | 14.6 (16.5) | 14.6 (19) | 25 (25) | >100 |
| **8** | 0.5 (0.5) | 14.6 (16.5) | 100 (>100) | 0.8 (0.8) | 54 (100) | 9.4 (12.5) | >100 |
| **9** | 0.6 (0.8) | 3.1 (7.8) | 20.8 (25) | 1.8 (2.3) | 4.2 (4.7) | 6.2 (6.2) | >100 |

| | | | | | | | |
|---|---|---|---|---|---|---|---|
| [a] Minimal inhibitory concentration (MIC) values in µM for ATC-peptides **1-9.** Minimal bactericidal concentration (MBCs) values in µM are indicated in bracket [b] dose required to lyse 50% of the red blood cells (HD50) are in µM. | | | | | | | |

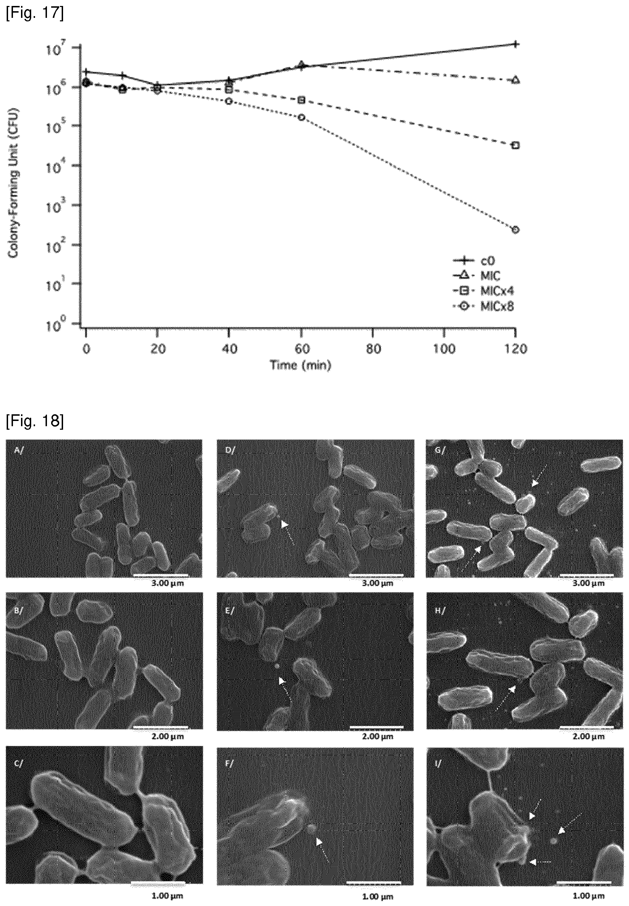

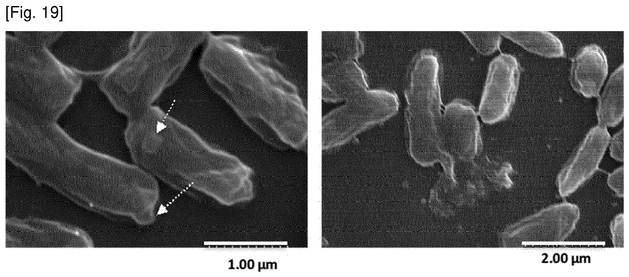

**Surface electron microscopy (SEM) experiments:** oligomer **9** was evaluated for its impact on *E. coli* morphology by surface electron microscopy (SEM). In order to define the best incubation period to observed antimicrobial effects, time-kill study was conducted over 2h incubation at 37°C. The average log reduction of the viable cell count ranged between 0.95 and 4.73 log₁₀ cfu/ml on incubating the bacteria for 2 h at the MIC and 8xMIC. Significant decrease of the growth rate was observed between 40 to 60 min at 4x and 8xMIC. TEM experiments were performed after 60 min contact between bacteria cells and compound 9. The untreated *E*. *coli* cells were about 2.0 µm long and displayed a smooth and intact surface (**Fig. 18A-C**). After incubation with 8xMIC of peptide **9,** the bacteria shortened to as little as 1.3 µm and appeared more compact indicating that the bacteria were not able to grow to maximum length. The cells exhibited increased region of cellular aggregation. Beside these changes of morphology, the cells showed blisters (**Fig. 19**) and protruding vesicles on their surface (**Fig. 18D-I**). In addition, whereas there were no fragments for control untreated bacteria, numerous spherical elements whose diameters were lower than 0.1µm were observed around bacteria, the number of which increasing with the peptide concentration. Despite some dispersion in size, such fragments, probable microsomes, were classically reported with other AMPs associated with membrane permeation and/or depolarization.[M. Hartmann, M. Berditsch, J. Hawecker, M. F. Ardakani, D. Gerthsen, A. S. Ulrich, Antimicrob. Agents Chemother. 2010, 54, 3132-3142*; b*/ E. Duval, C. Zatylny, M. Laurencin, M. Baudy-Floc'h, J. Henry, Peptides 2009, 30, 1608-1612.] Finally, at supra-MIC numerous lysed cells were observed (**Fig. 19**).

This work illustrates the potential of compounds of formula A as scaffold for the development of antibacterial and antifungal compounds.

### Procedure for Antimicrobial activity measurements :

the antimicrobial performances of oligomers antimicrobial activity were determined on three Gram positive strains (*Bacillus subtilis* ATCC6633, *Staphylococcus aureus* ATCC6538 and *Enterococcus faecalis* ATCC29121), two Gram negative strains (*Escherichia coli* ATCC8739 and *Pseudomonas aeruginosa* ATCC9027) and one fungal strain (*Candida albicans* ATCC10231). Each bacterial strain was allowed to grow overnight on Trypto-Caseine-Soja agar (TSA, Difco) at 37°C. *C. albicans* was allowed to grow 48h on a sabouraud medium (Difco) at 20-25°C. A fresh suspension at 10⁸ CFU.mL⁻¹ was then prepared by inoculating the micro-organisms in a Tryptone-Sel solution. Concentration was monitored by measuring transmitted light at 620 nm or with the Mac Farland turbidity standard (Mac Farland = 0.5 ≈ 10⁸ CFU.mL⁻¹). Each suspension was then diluted in a doubly strong Mueller-Hinton (MH, Difco) broth to 106 CFU.mL-1. Two-fold serial dilutions (100 µL) of oligomers in sterile water (pH adjusted between 7.4 and 7.7 with HCI and NaOH solutions) were mixed with microbial inoculum (100 µL) in NUNC sterile clear polystyrene flat-bottom 96-well plates. A 1/1 v/v oligomer solution/water mixture was used a negative control and a 1/1 vv water/microbial inoculum mixture was used a positive control. Plates were then incubated 24 h at 37°C for bacteria and 48 h at 25°C for *C. albicans.* Minimum Inhibitory Concentration (MIC) was determined as the lowest concentration inhibiting the growth of each micro-organism. After MIC determination, each well was transferred to a Petri dish containing MH agar, using a Mic-2000 inoculator (Dynatech) and was incubated 24 h at 37°C for bacteria and 48 h at 25°C for *C. albicans.* Minimum Bactericidal Concentration (MBC) was determined as the lowest concentration that prevent the growth of more than one colony. Each assay was performed in a triple duplicate for each strain.

**Time-kill study:** the antimicrobial kinetics of 9 were evaluated at 0, 1.8 µM (MIC), 7.2 µM (4xMIC) and 14.4 µM (8xMIC) and time intervals of 0, 10, 20, 40, 60 and 120 min (**Fig. 17**).

**Procedure for measurements of hemolytic activity:** Human red blood cells (hRBCs) were washed multiple times with phosphate-buffered saline (PBS) until a clear supernatant was obtained. The hRBCs suspension was made in PBS with a Packed Cell Volume of 5%. Two-fold serial dilutions (50 µL) of oligomers in sterile water (pH adjusted between 7.4 and 7.7 with HCI and NaOH solutions) were mixed with hRBCs suspension (50 µL) in clear polystyrene optical-bottom 96-well plates which were filmed and incubated for 1 h at 37°C. A 1/1 vv hRBCs solution/PBS mixture was used a negative control and a 1/1 vv hRBCs solution /1% (TRIS 50 mM pH= 8.0 + 1% SDS) mixture was used a positive control for 100% hemolysis. The mixture was then centrifuged at 1500g for 10 min at 5°C. Next, 50 µL of the supernatant was transferred into a second plate, and the absorbance was read at 490 nm using a BioRad iMark plate reader. The hemolysis percentage was calculated by the formula % hemolysis = [Absorbance (sample)-Absorbance (negative control)]/[Absorbance (positive control)- Absorbance (negative control)] x 100%. Each assay was performed in a double duplicate. Hemolytic activity was determined or estimated with the Hemolytic Concentration leading to 50% of lysis of hRBCs (HC50).

**Procedure for Surface electron microscopy:** PBS washed cells were fixed with 2.5% glutaraldehyde in PHEM buffer, pH 7.2 for an hour at room temperature, followed by washing in PHEM buffer. Fixed samples were dehydrated using a graded ethanol series (30-100%), followed by 10 minutes in graded Ethanol - Hexamethyldisilazane. And then Hexamethyldisilazane alone. Subsequently, the samples were sputter coated with an approximative 10nm thick gold film and then examined under a scanning electron microscope (Hitachi S4000, at CoMET,,MRI-RIO Imaging, Biocampus, INM Montpellier France) using a lens detector with an acceleration voltage of 10KV at calibrated magnifications.

## Claims

1. A compound of formula A: 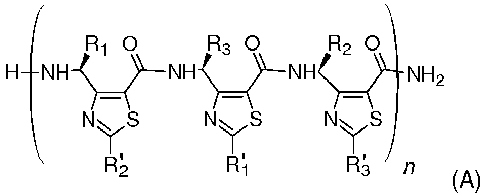
wherein n is an integer from 1 to 6,
wherein R₁ and R₁' are both either cationic residues or hydrophobic residues, said residues being identical or different
Wherein R₂ and R₂' are both either cationic residues or hydrophobic residues, said residues being identical or different ; and
Wherein R₃ and R₃' are both either cationic residues or hydrophobic residues, said residues being identical or different;
wherein
- when simultaneously R₁ and R₁' are cationic residues, then R₂, R₂', R₃ and R₃' are hydrophobic residues;
- when two simultaneously R₂ and R₂' are cationic residues, then R₁, R₁', R₃ and R₃' are hydrophobic residues; and
- when two simultaneously R₃ and R₃' are cationic residues, then R₁, R₁', R₂ and R₂' are hydrophobic residues,
or a salt or solvate thereof.

2. The compound according to claim 1, wherein said hydrophobic residues are chosen from the group consisting of : C₁-C₁₆ linear, branched or cyclic alkyl and C₄-C₁₆ aryl or arylalkyl, substituted of not, branched or not, or C₄-C₁₆ heteroaryl, or heteroarylaklyl substituted of not, branched or not.

3. The compound according to claim 1, wherein said cationic residue is a -(CH₂)m-R₄ residue, m being an integer from 1 to 7, wherein R₄ is an amino, an amido, an amidino, a guanino, a guanidino, an imino or a pyridino residue.

4. The compound according to anyone of claims 1 to 3, wherein when R₁ and R₁', or R₂ and R₂', or R₃ and R₃' are cationic residues, said residues being identical.

5. The compound according to anyone of claims 1 to 4, said compound being chosen from the compounds of the group consisting of : 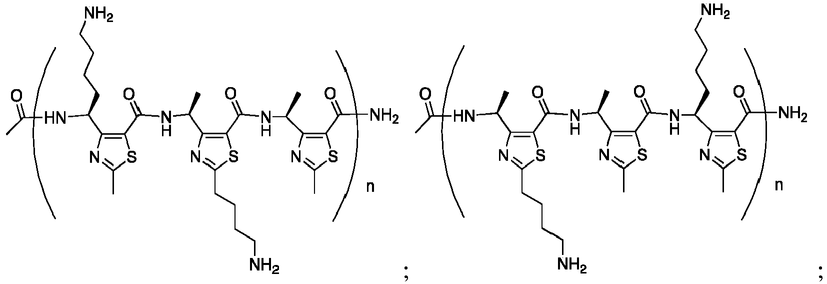 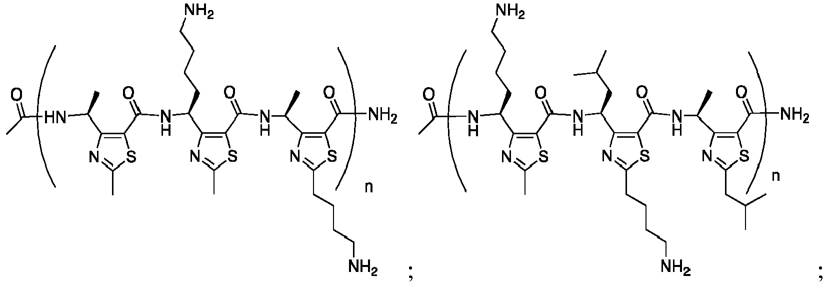 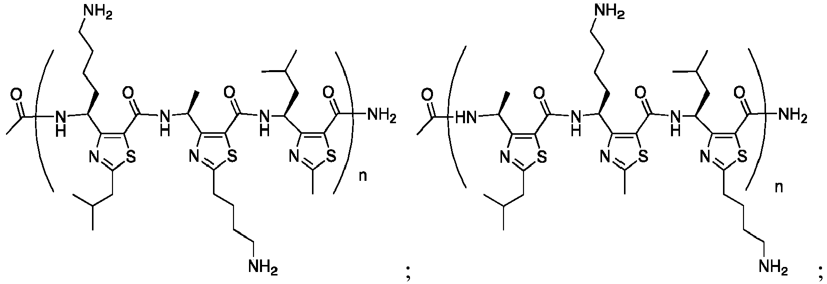 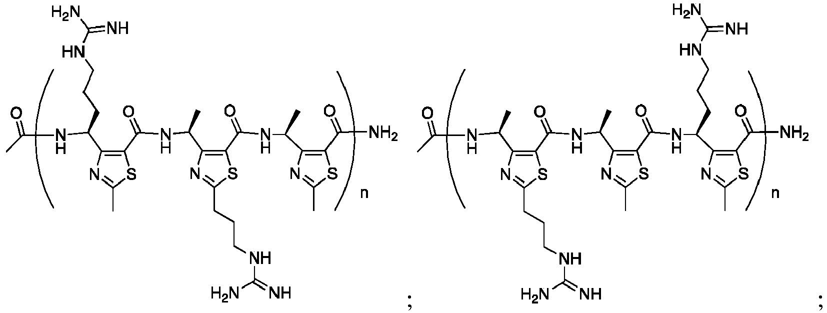 and

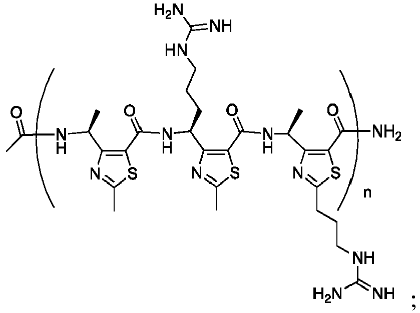

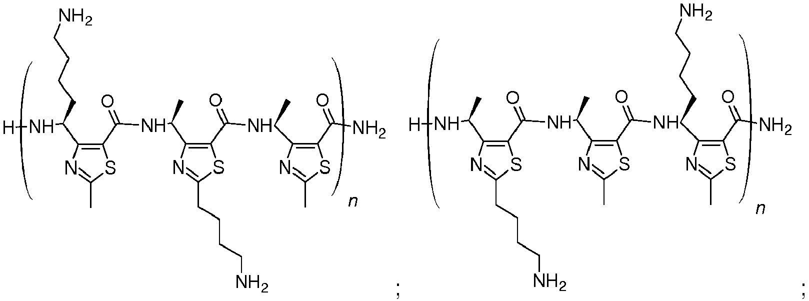

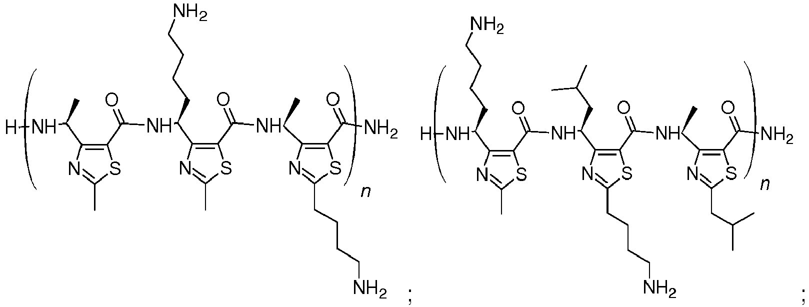

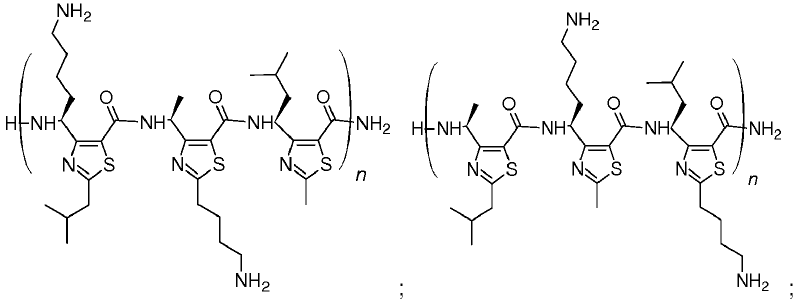

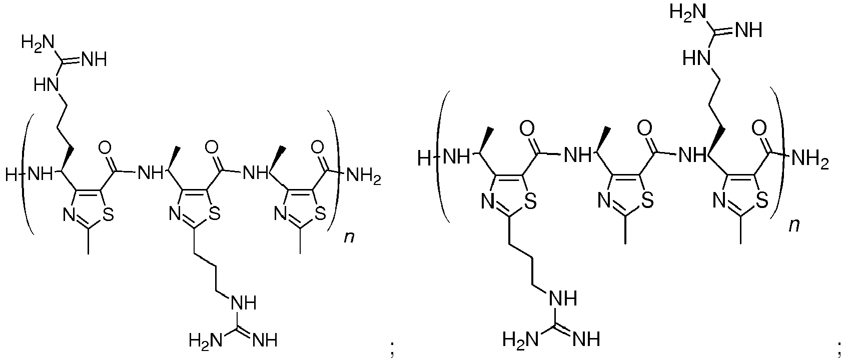

6. and 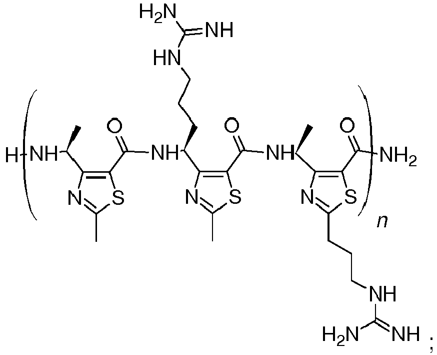 wherein n is an integer from 1 to 4.

7. The compound according to anyone of claims 1 to 5, wherein said compound is chosen from the compounds of the group consisting of :

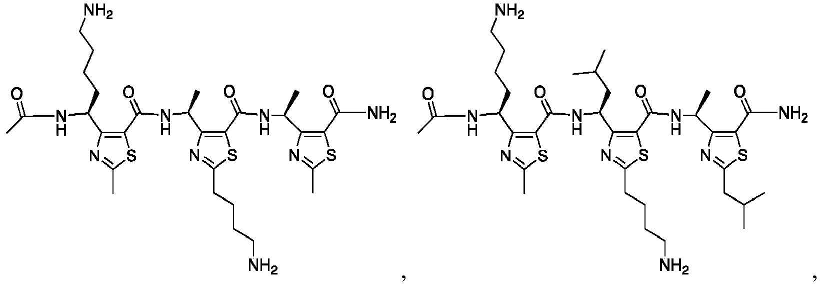

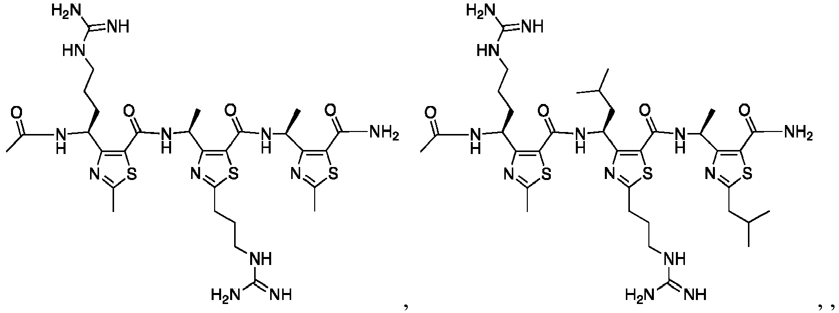

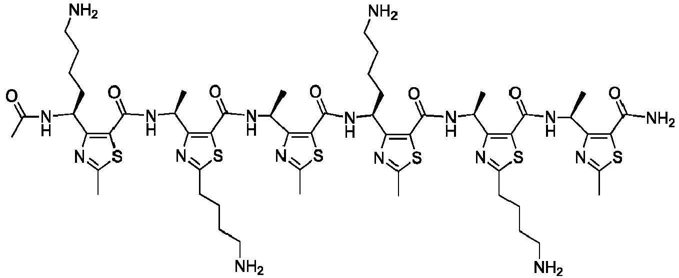

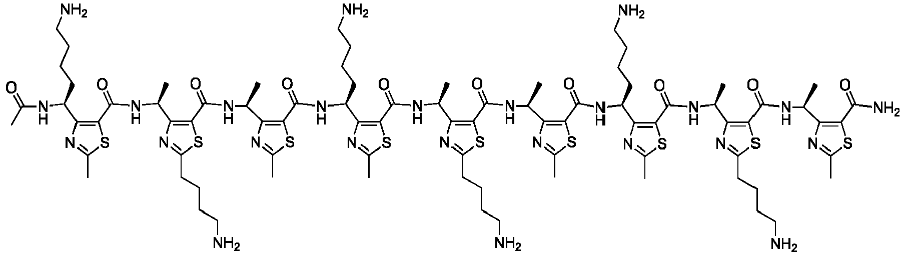

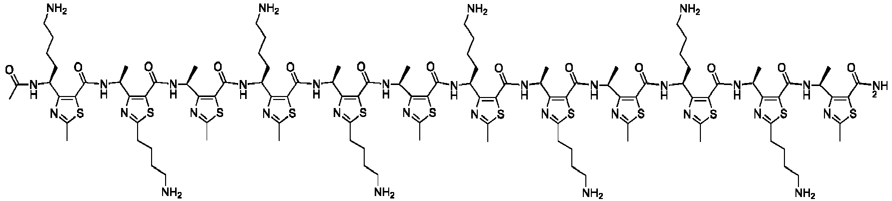

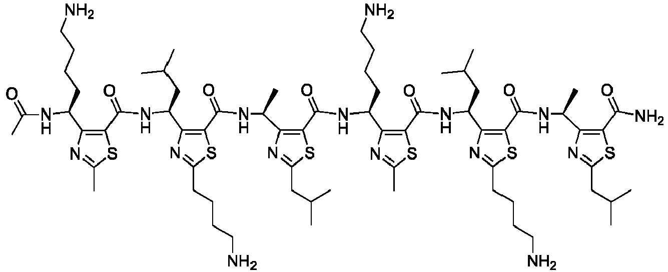

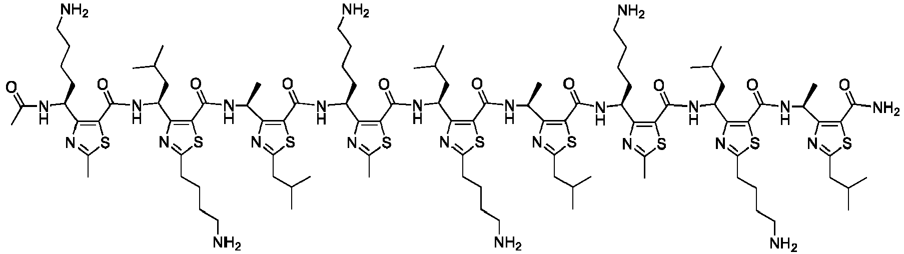

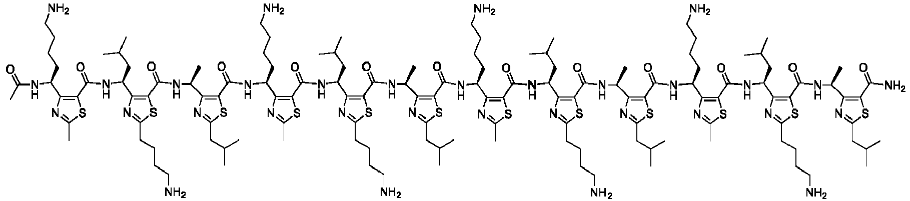

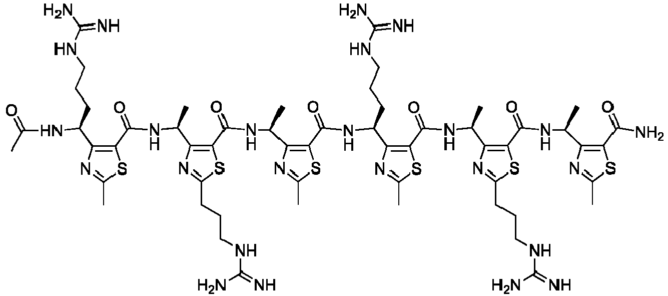

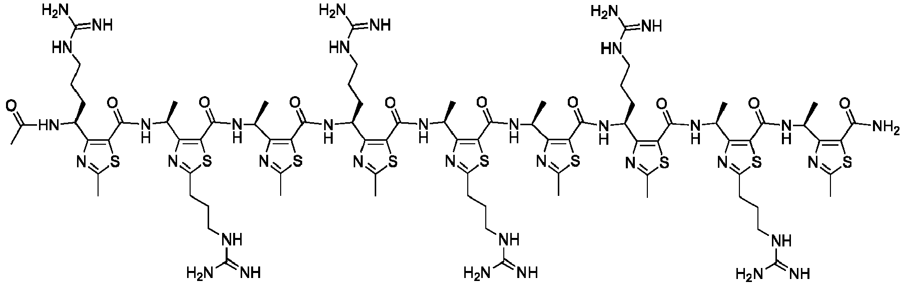

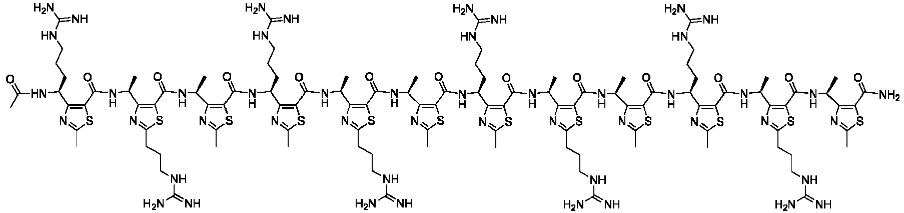

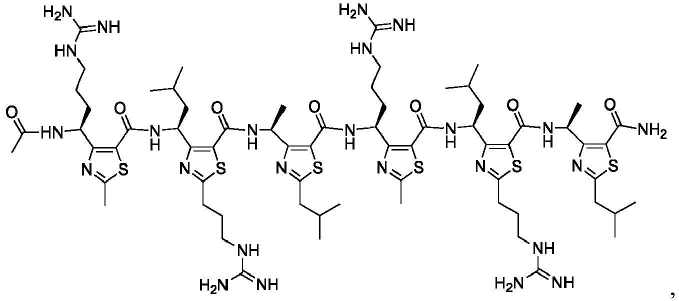

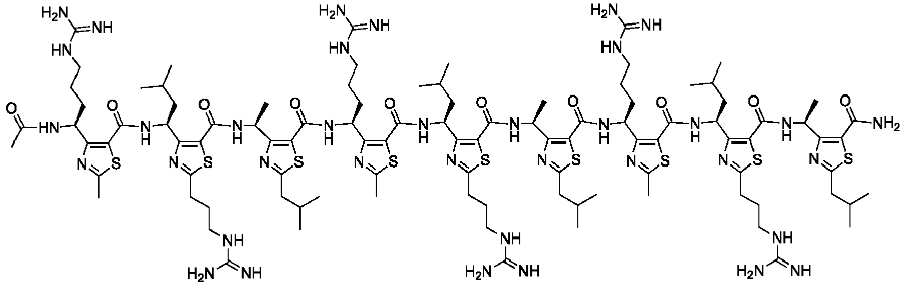

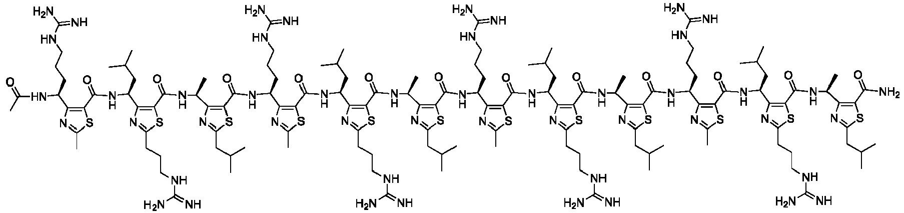

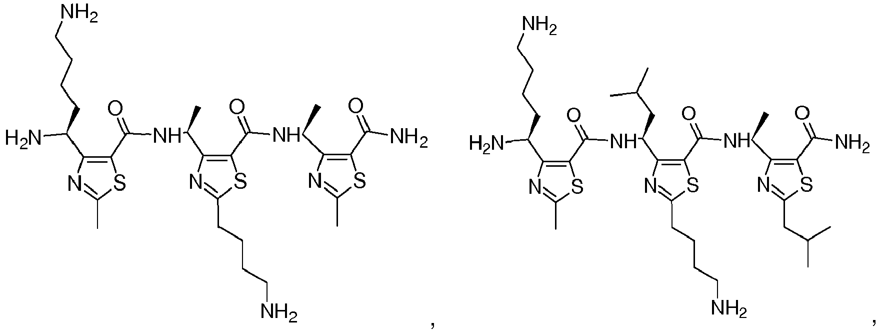

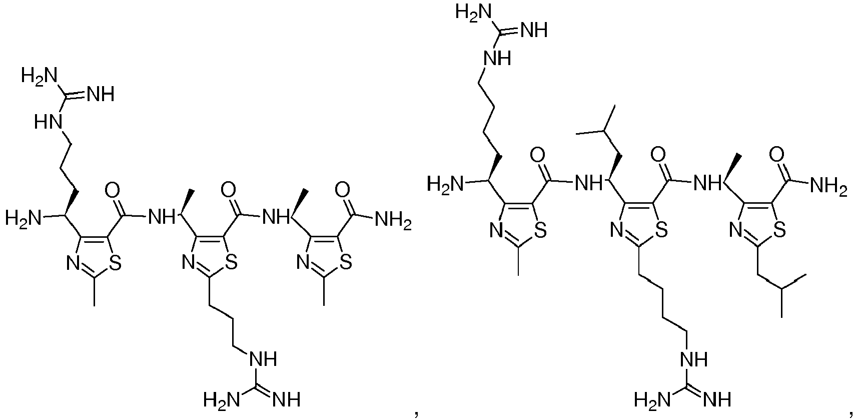

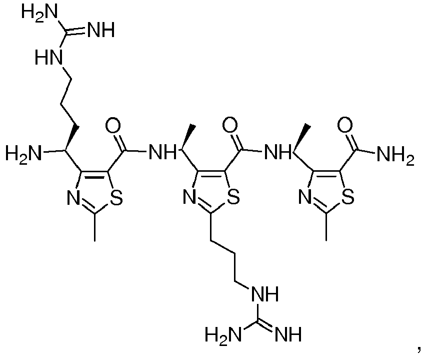

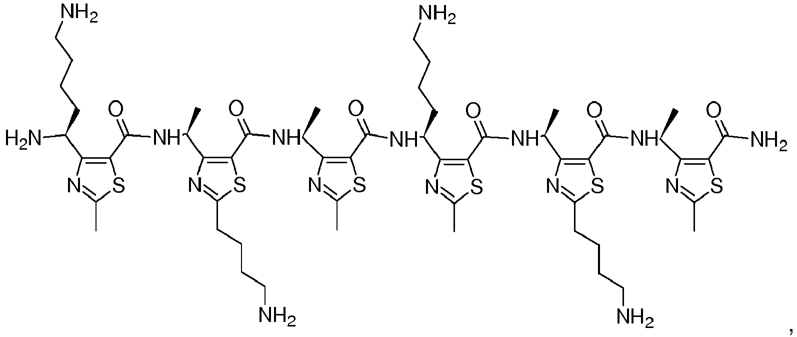

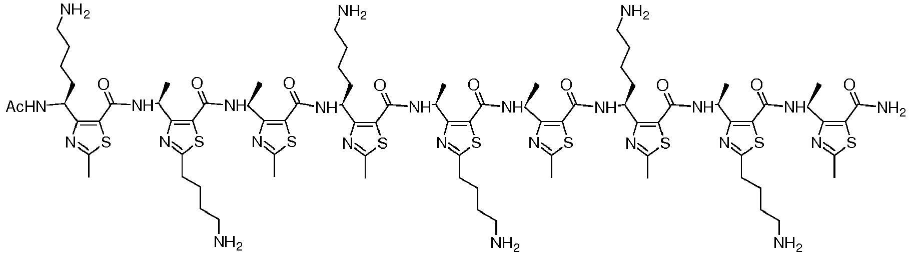

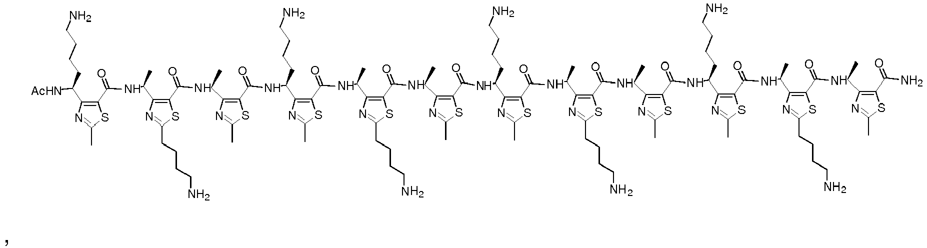

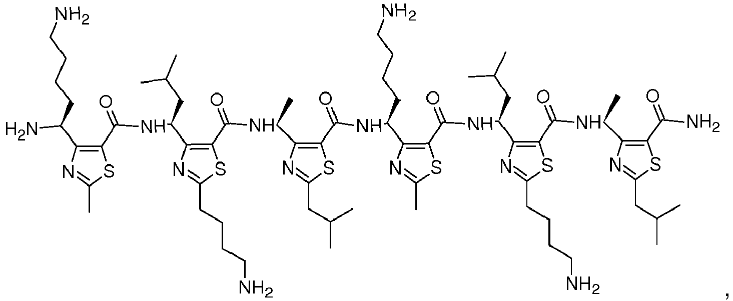

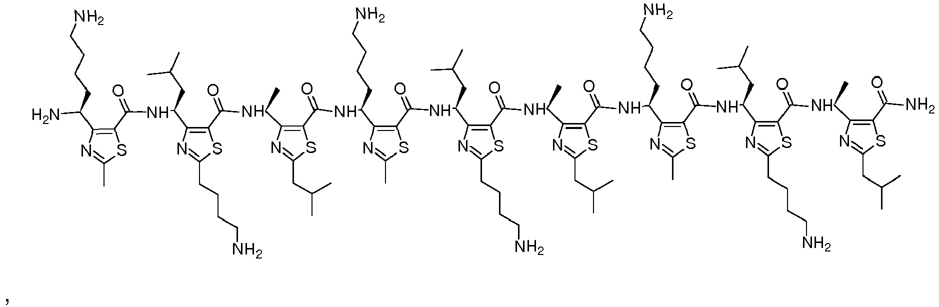

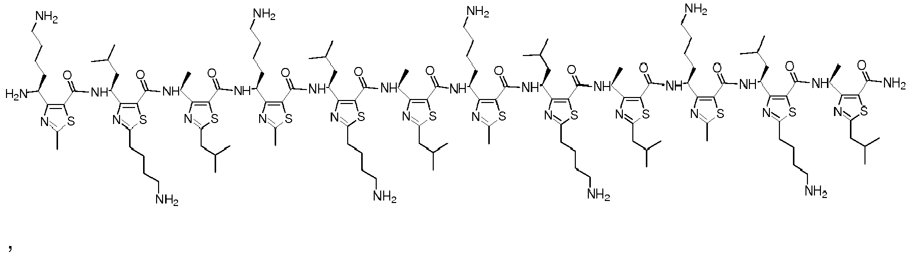

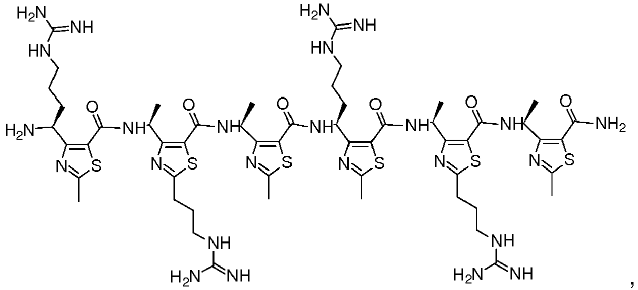

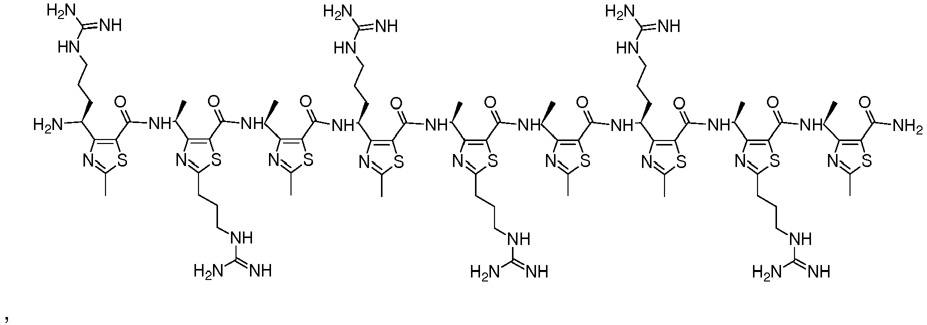

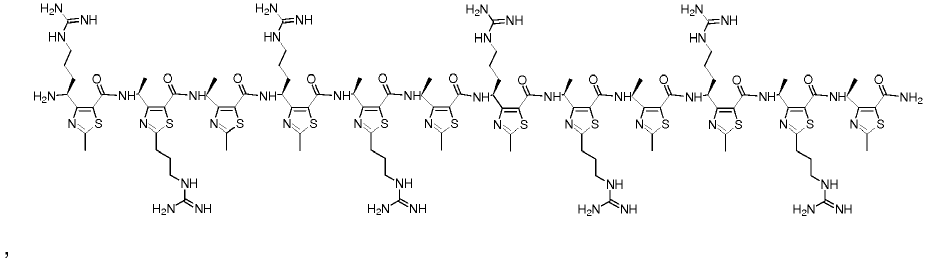

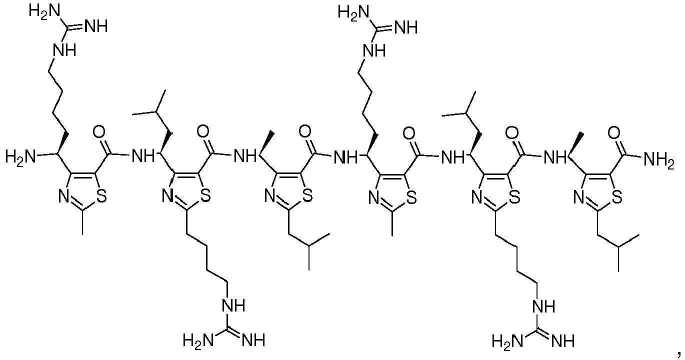

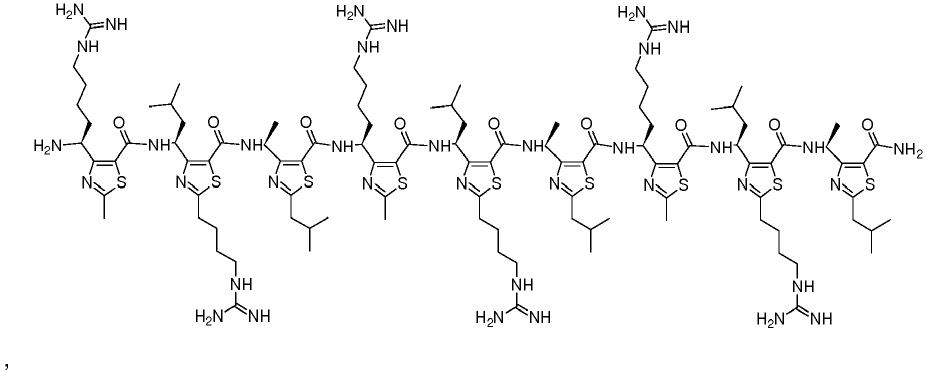

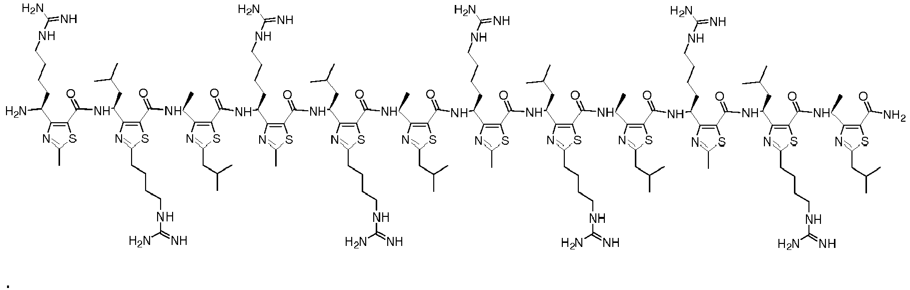

8. A pharmaceutical composition comprising as active ingredient a compound according to anyone of claims 1 to 6, in association with a pharmaceutically acceptable carrier.

9. A compound according to anyone of claims 1 to 6, for its use as a medicine.

10. A compound according to anyone of claims 1 to 5, for its use for the treatment of infection caused by bacteria and fungi.

11. Use of a compound according to anyone of claims 1 to 6, for the decontamination of a surface infected by bacteria and fungi.

12. Compound of formula **B** : 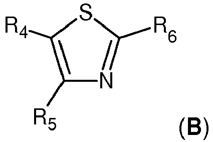 wherein R₄ is a C₁-C₄ acid, R₅ and R₆ are, independently from each other a C₁-C₅, linear or branched, alkyl, a C₁-C₅, linear or branched, alkyl, substituted by a diamine imine, protected or not, or a C₁-C₅, linear or branched, alkyl, substituted by an amine, protected or not.

13. Compound according to claim 12, wherein said compound is chosen from the compounds of the group consisting of : 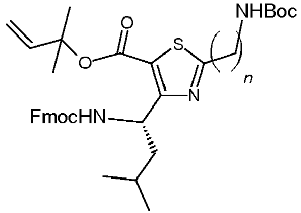 wherein n = 4, 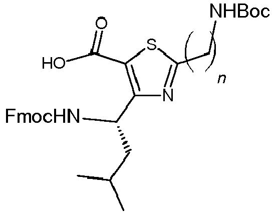 wherein n = 4, 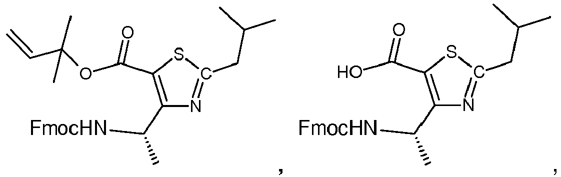 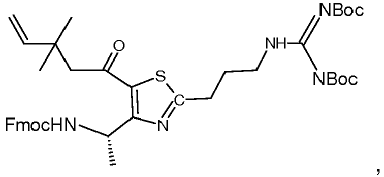 and
